# EUROPEAN PATENT SPECIFICATION

(11) **EP 4 386 760 B1**
(45) Date of publication and mention of the grant of the patent: **12.11.2025**
(21) Application number: 22306858.6
(22) Date of filing: 13.12.2022
(51) Int. Cl.: G16B 20/20, G16B 5/10, G16B 40/20, G16H 50/20

(54) **DEVICE AND METHOD FOR DECISION SUPPORT IN STANDARDIZED PHENOTYPING**
VORRICHTUNG UND VERFAHREN ZUR ENTSCHEIDUNGSUNTERSTÜTZUNG BEI DER STANDARDISIERTEN PHÄNOTYPISIERUNG
DISPOSITIF ET PROCÉDÉ D'AIDE À LA DÉCISION DANS UN PHÉNOTYPAGE STANDARDISÉ

(43) Date of publication of application: 19.06.2024
(73) Proprietor: Seqone, 34000 Montpellier (FR)
(72) Inventor: BEAUMEUNIER, Sacha, 34540 Balaruc le Vieux (FR); YAUY, Kevin, 34670 Montpellier (FR); LARUE, Dimitri, 34170 Castelnau le Lez (FR); PHILIPPE, Nicolas, 34150 Gignac (FR); AUDOUX, Jérome, 34000 Montpellier (FR); DUFORET, Nicolas, 34670 Saint Bres (FR)
(74) Representative: Icosa

(56) References cited:
- WO-A1-2019/169452
- WO-A1-2022/212337
- CHEN ZEFU ET AL: "PhenoApt leverages clinical expertise to prioritize candidate genes via machine learning", THE AMERICAN JOURNAL OF HUMAN GENETICS, AMERICAN SOCIETY OF HUMAN GENETICS , CHICAGO , IL, US, vol. 109, no. 2, 20 January 2022 (2022-01-20), pages 270 - 281, XP086950046, ISSN: 0002-9297, [retrieved on 20220120], DOI: 10.1016/J.AJHG.2021.12.008
- ZHAO MENGGE ET AL: "Phen2Gene: Rapid Phenotype-Driven Gene Prioritization for Rare Diseases", BIORXIV, 10 December 2019 (2019-12-10), XP055901742, Retrieved from the Internet <URL:https://www.biorxiv.org/content/10.1101/870527v1.full.pdf> [retrieved on 20220315], DOI: 10.1101/870527

## Description

### FIELD OF INVENTION

The present invention relates to methods and devices for genomic analysis. More in details, the present invention relates to methods and devices for the calculation of a symptom-gene and symptom-symptom association atlas and its utilization for standardization of clinical description and for the identification of one or more gene(s) that has the highest probability to be at the origin of a disease manifested by a subject.

### BACKGROUND OF INVENTION

Precision medicine relies on patient stratification and recognition of clinically relevant groups to improve diagnosis, prognosis, and medical treatment. Phenotyping allows homogeneous groups of individuals to be constituted, where physicians report characteristics deviating from normal morphology, physiology, and behavior using standardized descriptions in the Human Phenotype Ontology (HPO)^{2,3}. Despite a common ontology and abundant clinical data, medical records often lack consistency and comparability between descriptions and practitioners, which is referred to as fuzzy matching in phenotype profiles⁴. This inconsistent phenotyping is a major hurdle to fully exploiting the clinical data contained in medical records.

Identical symptoms observed in patients may heterogeneously be described by physicians, even though relying on a predefined ontology, such as the Human Phenotype Ontology (HPO). In particular, the inventors carried out a study, further detailed in the present disclosure, about current phenotyping practices, showing this heterogeneity. Current algorithms address phenotyping heterogeneity using the ontology structure either to extract additional symptom-gene associations from literature or to evaluate the semantic similarity of symptoms.

Document WO2019169452A1 discloses a system and method for creating interactive graphical visualization of clinical data of observed phenotypes of a patient and genetic data. A database is accessed to determine associations of observed phenotypes with disorders and a second database is accessed to determine associations between the disorders and genetic properties. The processor then determines an association value for each combination of the observed phenotypes and the genetic properties based on a number of paths between them. The processor also generates a graphical user interface, comprising an arrangement of the association values and a user control element associated with the phenotypes and/or the genetic properties.

In contrast to these approaches, the inventors of the present invention used HPO as a dictionary of symptoms and considered relationships between symptoms only through their co-occurrence in genetic diseases found in HPO-structured and text-mined databases.

In this context, the present disclosure proposes a solution that allows to fill the gaps in the currently available database.

### SUMMARY

An aspect of the disclosure relates to a device for the identification of genes having the highest probability to be associated to symptoms observed for a subject, said device comprising:
- at least one input adapted to receive subject data representative of at least one symptom observed for the subject;
- at least one processor configured to:
   - associate each subject data representative of one symptom of the subject to one phenotype *j* represented in a gene-phenotype matrix; said gene-phenotype matrix being representative of associations between at least one phenotype and at least one gene considered to be responsible of said associated at least one phenotype, wherein each coefficient *aᵢⱼ* of said gene-phenotype matrix represents a probability of association between gene *i* and phenotype *j*;
   - calculate a subject specific matrix comprising as columns each column of the gene-phenotype matrix for the phenotype *j* associated to the at least one symptom of the subject;
   - calculate a score vector, wherein each coefficient *bᵢ* of the score vector is obtained as function of each row coefficients of the subject specific matrix so as to be representative of the probability that each gene of the subject specific matrix is associated to the symptoms observed for the subject;
- at least one output adapted to provide at least one prediction based on said score vector, said prediction providing an information of the gene(s) that is(are) more likely to be associated to the symptoms observed for the subject.

The present disclosure advantageously provides a device (and a method discussed here below) allowing an accurate calculation of the probabilities that different genes are at the origin of a disease of the subject, which allows to provide an accurate ranking of the genes. Said ranking is obtained in a very fast manner as it is the result of a simple set of operations on a numeric matrix (i.e., the gene-phenotype matrix).

In one embodiment, the at least one processor is configured to:
- calculate a pathogenic vector wherein each coefficient is representative of a frequency at which a variant of each gene *i* is reported as pathogenic in a predefined medical database; and
- subtract to each coefficient *bᵢ* of the score vector the coefficient of the pathogenic vector obtained for the gene *i*.

According to one embodiment, the at least one processor is configured to:
- calculate a correction vector so as to take into account missing phenotypes associated to gene *i*, wherein each coefficient of said correction vector is calculated as the sum of the coefficient of one row of the subject-specific matrix associated to the gene *i*; and

subtract to each coefficient *bᵢ* of the score vector associated coefficient of the correction vector obtained for the gene *i*.

According to one embodiment, the at least one processor is further configured to calculate the gene-phenotype matrix by:
- defining a matrix *N* × M wherein *N* is number of genes and *M* is the number of phenotypes present in a list comprising associations between phenotypes and genes; the coefficients *aᵢⱼ* of the gene-phenotype matrix (being set to a predefined maximum non-zero value if the list comprises an association between said gene *i* and said phenotype *j* or otherwise to zero;
- for each coefficient *aᵢⱼ* relating to an association of the gene *i* and the phenotype *j* absent from said list, calculating the value of said coefficient *aᵢⱼ* as function of a distance between the phenotype *j* and the closest phenotype for the gene i, whose association is present in the list; wherein the distance between the phenotype *j* and the closest phenotype is calculated on a direct acyclic graph representative of a standard categorization of the human abnormal phenotypes and of their semantic relationships.

According to one embodiment, the at least one processor is further configured to calculate the gene-phenotype matrix by:
- calculate at least two intermediate gene-phenotype matrices, each from a respective list comprising associations between at least one phenotype and at least one gene;
- obtain the gene-phenotype matrix as a linear combination of the calculated intermediate gene-phenotype matrices.

According to one embodiment, for the calculation of the gene-phenotype matrix, the at least one processor is further configured to apply a normalization weight to each column j of the gene-phenotype matrix, wherein the weight w(j) is function of the frequency of appearance of the phenotype j with respect to the genes represented in the gene-phenotype matrix.

According to one embodiment, the at least one processor is further configured to rank the genes associated to the symptoms observed in the subject on the base of the base of the score vector.

The present disclosure further relates to a computer-implemented method for the identification of genes having the highest probability to be associated to symptoms observed for a subject, said method comprising:
- receiving subject data representative of at least one symptom observed for the subject;
- associating each subject data representative one symptom of the subject to one phenotype j represented in a gene-phenotype matrix; said gene-phenotype matrix being representative of associations between a symptom and at least one gene considered to be responsible of said associated symptom, wherein each coefficient *aᵢⱼ* of said gene-phenotype matrix represents a probability of association between gene *i* and phenotype *j*;

- calculating a subject specific matrix comprising as columns each column for the phenotype *j* associated to the at least one symptom of the subject;
- calculating a score vector wherein each coefficient *bᵢ* of the score vector is obtained as function of each row coefficients of the subject specific matrix so as to be representative of the probability that has each gene, represented in the subject specific matrix, to be associated to the symptoms observed for the subject;
- outputting at least one prediction based on said the score vector, said prediction providing an information of the gene(s) that is(are) more likely to be associated to the symptoms observed for the subject.

According to one embodiment, the method further comprises:
- calculating a pathogenic vector wherein each coefficient is representative of a frequency at which a variant of each gene *i* is reported as pathogenic in a predefined medical database; and
- subtracting to each coefficient *bᵢ* of the score vector the coefficient of the pathogenic vector obtained for the gene *i*.

According to one embodiment, the method further comprises calculating the gene-phenotype matrix by:
- defining a matrix *N* × M wherein *N* is number of genes and *M* is the number of phenotypes present in a first list comprising associations between phenotypes and genes; the coefficients *aᵢⱼ* of the gene-phenotype matrix (21) being set to a predefined maximum non-zero value if the list comprises an association between said gene i and said phenotype *j* or otherwise to zero;
- for each coefficient *aᵢⱼ* relating to an association of the gene *i* and the phenotype *j* absent from said first list, calculating the value of each coefficient *aᵢⱼ* as function of a distance between the phenotype *j* and the closest phenotype for the gene i, whose association is present in the first list; wherein the distance between the phenotype j and the closest phenotype is calculated on a direct acyclic graph representative of a standard categorization of the human abnormal phenotypes and of their semantic relationships.

The present disclosure relates also to a device for calculating a gene-phenotype matrix to be used as genotype-phenotype atlas in a method for the identification of genes having the highest probability to be associated to symptoms observed for a subject according to the present invention; said device comprising:
- at least one input adapted to receive a list comprising associations between phenotypes and genes;
- at least one processor configured to:
   - defining a matrix *N* × *M* wherein *N* is number of genes and *M* is the number of phenotypes present in a list comprising associations between phenotypes and genes; the coefficients *aᵢⱼ* of the gene-phenotype matrix being set to a predefined maximum non-zero value if the list comprises an association between said gene i and said phenotype *j* or otherwise to zero;
   - for each coefficient *aᵢⱼ* relating to an association of the gene i and the phenotype *j* absent from said list, calculating the value of said coefficient *aᵢⱼ* as function of a distance between the phenotype *j* and the closest phenotype for the gene *i*, whose association is present in the list; wherein the distance between the phenotype j and the closest phenotype is calculated on a direct acyclic graph representative of a standard categorization of the human abnormal phenotypes and of their semantic relationships.
- at least one output adapted to provide at least one gene-phenotype matrix representative of associations between at least one phenotype and at least one gene considered to be responsible of said associated at least one phenotype.

According to one embodiment, the at least one processor of the device for calculating the gene-phenotype matrix is further configured to calculate the gene-phenotype matrix by:
- calculate at least two intermediate gene-phenotype matrices, each from a respective list comprising associations between at least one phenotype and at least one gene;
- obtain the gene-phenotype matrix (21) as a linear combination of the calculated intermediate gene-phenotype matrices.

According to one embodiment, the at least one processor of the device for calculating the gene-phenotype matrix is further configured to apply a normalization weight to each column j of the gene-phenotype matrix, wherein the weight w(j) is function of the frequency of appearance of the phenotype j with respect to the genes represented in the gene-phenotype matrix.

The present disclosure also relates to the method for generating the gene-phenotype matrix according to the device for calculating the gene-phenotype matrix described hereabove. Said gene-phenotype matrix is an advantageously data structure that is easier to use for fast computation of gene prioritization, while integrating the information from many different sources or thesaurus. Indeed, all information is embodied in one numerical matrix.

In addition, the disclosure relates to a computer program comprising software code adapted to perform a method for the identification of genes or a method for gene-phenotype matrix calculation compliant with any of the above execution modes when the program is executed by a processor.

The present disclosure further pertains to a non-transitory program storage device, readable by a computer, tangibly embodying a program of instructions executable by the computer to perform a method for the identification of genes or a method for gene-phenotype matrix calculation, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

Another aspect of the invention relates to a computer-implemented method for decision support of a user to standardize phenotyping in genomic analysis of a subject, wherein the method comprises:
- a) receiving a list of symptoms comprising at least one symptom observed for the subject, wherein the list of symptoms comprises an integer number N of symptoms;
- if the integer number N is higher than a threshold value, the method further comprises a step b1) of receiving a first graph comprising nodes and weighted links, wherein:
   o each node is representative of one symptom, each symptom being associated to at least one gene;
   o each link connecting two nodes is representative of a similarity between the two symptoms associated to the two connected nodes, and
   o the weight of each link represents a degree of similarity between said two symptoms associated to the two connected nodes;
   said first graph being previously obtained calculating symptoms pair similarities using information concerning associations between at least one symptom and at least one gene considered to be responsible of said associated at least one symptom;
- else if the integer number is lower than or equal to said threshold value, the method further comprises steps b21), b22), b23) and b24) comprising:
   - b21) receiving a second graph being previously obtained by applying a matrix factorization to a gene-symptom matrix, said second graph comprising nodes and weighted links, wherein each node is representative of one symptom, each symptom being associated to at least one gene; each node is associated to at least one group of nodes of the second graph; and each node is associated to at least one score of specificity, each score of specificity representing the specificity of the symptom associated to the node with respect to the symptoms of the nodes in each group to which is associated said node; wherein said gene-symptom matrix is representative of associations between at least one symptom and at least one gene considered to be responsible of said associated at least one symptom, wherein each coefficient *aᵢⱼ* of said gene-symptom matrix represents a probability of association between gene i and symptom j;
      wherein each gene represented in the gene-symptom matrix is associated to each group of nodes of the second graph by a gene-group weighting score;
   - b22) evaluating the specificity of the received list of symptoms, using the score of specificity of the node associated the at least one symptom in the second graph, and if the specificity of the list of symptoms does not satisfy a predefined rule, providing to the user a suggestion of at least one new symptom; each at least one suggested new symptom being obtained as the symptom associated to the node in the first graph connected by one link to the node associated to the at least one symptom of the received list of symptoms;
   - b23) if the at least one suggested new symptom is added to the received list of symptoms by the user, evaluating the specificity of the updated list of symptoms comprising the at least one received symptom and the at least one new suggested symptom, using the score of specificity of the node in the second graph associated to the symptoms in the updated list of symptoms;
   - b24) whenever the specificity of the list of symptoms satisfies the predefined rule, using said list of symptoms and the first graph to obtain at least one gene having the highest probability to be associated to the list of symptoms;
      the method further comprising the following steps:
      - c) if, after providing to the user a suggestion of at least one new symptom, the specificity of the updated list of symptoms does not satisfy the predefined rule:
         - generating a symptoms binary vector representing the symptoms in the list of symptoms and applying the matrix factorization to said binary vector so as to obtain a representation of the updated list of symptoms on the groups of the second graph, said representation comprising one patient score for each group of the second graph;
         - calculating a difference between each patient score and each gene-group weighting score for each group of the second graph;
         - normalizing said differences with respect to a reference set of distances representative of a subject without symptoms;
         - selecting at least one gene having the highest probability to be associated to the updated list of symptoms as the at least one gene having the highest normalized difference;
   - d) outputting the at least one gene associated to the list of symptoms or the updated list of symptoms.

Thanks to the method according to this aspect of the invention, it is possible to handle heterogeneous phenotyping by developing symptom interaction models, represented by the first graph and the second graph, to standardize clinical descriptions, and, based on those standardized clinical descriptions, to identify genes having the highest probability to be associated to symptoms observed for a subject.

The second graph allows indeed associating one symptom among the list of symptoms observed for the subject to one group of symptoms representing a phenotypic pattern in genetic diseases. Thanks to the first graph, which links symptoms with a given similarity, the list of symptoms may be enriched in order to help determine the genes the most probable to be associated to the symptoms observed for the subject.

In an embodiment, the threshold value is equal to 5.

Another aspect of the invention relates to a device for decision support of a user to standardize phenotyping in genomic analysis of a subject, wherein the device comprises:
- at least one input configured to receive:
   o a list of symptoms comprising at least one symptom observed for the subject, wherein the list of symptoms comprises an integer number N of symptoms;
   o a first graph comprising nodes and weighted links, wherein each node is representative of one symptom, each symptom being associated to at least one gene; each link connecting two nodes is representative of a similarity between the two symptoms associated to the two connected nodes, and the weight of each link represents a degree of similarity between said two symptoms associated to the two connected nodes;
   said first graph being previously obtained calculating symptoms pair similarities using information concerning associations between at least one symptom and at least one gene considered to be responsible of said associated at least one symptom;
   o a second graph being previously obtained by applying a matrix factorization to a gene-symptom matrix, said second graph comprising nodes and weighted links, wherein each node is representative of one symptom, each symptom being associated to at least one gene; each node is associated to at least one group of nodes of the second graph; and each node is associated to at least one score of specificity, each score of specificity representing the specificity of the symptom associated to the node with respect to the symptoms of the nodes in each group to which is associated said node; wherein said gene-symptom matrix is representative of associations between at least one symptom and at least one gene considered to be responsible of said associated at least one symptom, wherein each coefficient *aᵢⱼ* of said gene-symptom matrix represents a probability of association between gene i and symptom j; and wherein each gene represented in the gene-symptom matrix is associated to each group of nodes of the second graph by a gene-group weighting score;
- at least one processor configured to:
   ∘ if the integer number N is less than or equal to a threshold value:
      ▪ evaluate a specificity of the list of symptoms, using the score of specificity of the node associated the at least one symptom in the second graph, and if the specificity of the list of symptoms does not satisfy a predefined rule, providing to the user a suggestion of at least one symptom, each at least one suggested symptom being obtained as the symptom associated to the node in the first graph connected by one link to the node associated to the at least one received symptom;
      ▪ if the at least one suggested symptom is added to the list of symptoms by the user, evaluate the specificity of the list of symptoms comprising the at least one received symptom and the at least one suggested symptom, using the score of specificity of the node in the second graph associated to the symptoms in the list of symptoms;
      ▪ whenever the specificity of the list of symptoms satisfies the predefined rule, use said list of symptoms and the first graph to obtain at least one gene having the highest probability to be associated to the list of symptoms;
   o if, after providing to the user a suggestion of at least one symptom, the specificity of the list of symptoms does not satisfy a predefined rule:
      ▪ generate a symptoms binary vector representing the symptoms in the list of symptoms and applying the matrix factorization to said binary vector so as to obtain a representation of the list of symptoms on the groups of the second graph, said representation comprising one patient score for each group of the second graph;
      ▪ calculate a difference between each patient score and each gene-group weighting score for each group of the second graph;
      ▪ normalize said differences with respect to a reference set of distances representative of a subject without symptoms;
      ▪ select at least one gene having the highest probability to be associated to the list of symptoms as the at least one gene having the highest normalized difference;
- at least one output configured to provide the at least one gene associated to the list of symptoms.

In one or more embodiments, the matrix factorization is a non-negative matrix factorization.

Thus, as the second graph is obtained by factorizing a gene-symptom matrix by means of a non-negative matrix factorization, the obtention of the at least one group of nodes of the second graph is easier to obtain. This is due to the inherent clustering property of the non-negative matrix factorization method.

In one or more embodiments, the non-negative matrix factorization applied to the gene-symptom matrix is a Non-negative Double Singular Value Decomposition (NNDSVD) initialization. The use of the NNDSVD initialization technique renders the non-negative matrix factorization process more efficient and faster.

In one or more embodiments, information concerning associations between at least one symptom and at least one gene is a gene-symptom list representative of associations between at least one symptom and at least one gene considered to be responsible of said associated at least one symptom or is the gene-symptom matrix.

In one or more embodiments, the weight of each link in the first graph is determined with a node similarity algorithm.

As the weight of each link represents a degree of similarity between two symptoms associated to the two connected nodes, the node similarities algorithm allows, by comparing a set of nodes based on the nodes they are connected to, computing pair-wise similarities based on an Overlap coefficient. Each weight of a link is one computed pair-wise similarity.

In one or more embodiments, the using of the list of symptoms and the first graph to obtain the at least one gene having the highest probability to be associated to the list of symptoms comprises:
a. determining at least one additional symptom associated to at least one symptom of the list of symptoms by identifying, among the nodes of the first graph, at least one node connected, by a weighted link having a weight higher than a predefined threshold, to one of the at least one node corresponding to the symptoms on the list of symptoms;
b. calculating a subject specific matrix comprising as columns each column of the gene-phenotype matrix for the symptom j associated to the at least one symptom of the subject comprised in the list of symptoms and said at least one additional symptom;
c. calculating a score vector, wherein each coefficient *bᵢ* of the score vector is obtained as function of each row coefficients of the subject specific matrix so as to be representative of the probability that each gene of the subject specific matrix is associated to the symptoms observed for the subject;
d. using the score vector to obtain an information on the at least one gene that is more likely to be associated to the symptoms observed for the subject.

Thus, thanks to the first graph, it is possible to enrich the list of symptoms with the at least one additional symptom, determined on the basis of a similarity with symptoms of the list of symptoms, to help the process of determining information on the at least one gene more likely to be associated to the symptoms observed for the subject.

In one or more embodiments, the at least one processor is further configured to rank the genes associated to the symptoms observed in the subject on the base of the score vector.

### DEFINITIONS

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The terms "electronic heath record" refers digital version of a patient's paper chart. EHRs are real-time, patient-centered records that make information available instantly and securely to authorized users. Contain a patient's medical history, diagnoses, medications, treatment plans, immunization dates, allergies, radiology images, and laboratory and test results.

As used herein, the term **"subject"** refers to a mammal, preferably a human. In one embodiment, a subject may be a **"patient",** *i.e.,* a warm-blooded animal, more preferably a human, who/which is awaiting the receipt of, or is receiving medical care or was/is/will be the object of a medical procedure, or is monitored for the development of a disease, such as cancer. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The term **"symptoms"** relates to information derived from observations or measures made on the subject (i.e., blood pressure and other information such as weight, body dimension), laboratory and test results such as genome analysis, blood analyses and the like.

The term **"phenotype"** relates to any observable characteristic or symptom of a disease, such as morphology, development, biochemical or physiological properties, or behavior, without any implication of a mechanism. A clinical phenotype would be the presentation of a disease in a given individual. A medical ontology, such as the Human Phenotype Ontology (HPO), provides standardized vocabulary of phenotypic abnormalities encountered in human disease, that may be used to associate the observable characteristic or symptoms to corresponding phenotypes.

The term **"panel of genes"** refers to a test that analyzes multiple genes at once for a specific clinical indication.

### BRIEF DESCRIPTION OF THE DRAWINGS

**Figure 1** is a block diagram representing schematically a particular mode of a device for gene prioritization, compliant with the present disclosure.
**Figure 2** is a block diagram representing schematically a second mode of the device for gene prioritization, compliant with the present disclosure.
**Figure 3** is a flow chart showing successive steps executed with the device for predicting of figure 1.
**Figure 4** is a flow chart showing successive steps executed with the device for predicting of figure 2.
**Figure 5** diagrammatically shows an apparatus integrating the functions of the device for prioritization of figure 1.
**Figures 6A-6C** are a combination of graphs illustrating a landscape of phenotyping practices from a retrospective cohort of 1,686 patients and a prospective experiment of clinical reports phenotyped by multiple physicians. **Fig. 6A** shows a treemap chart of the HPO terms frequency across the retrospective cohort. **Fig. 6B** shows aiolin plot of HPO term counts per clinical description for each subgroup of the cohort. **Fig. 6C** shows a violin plot of HPO term counts per clinical description for each clinical report phenotyped by 12 physicians.

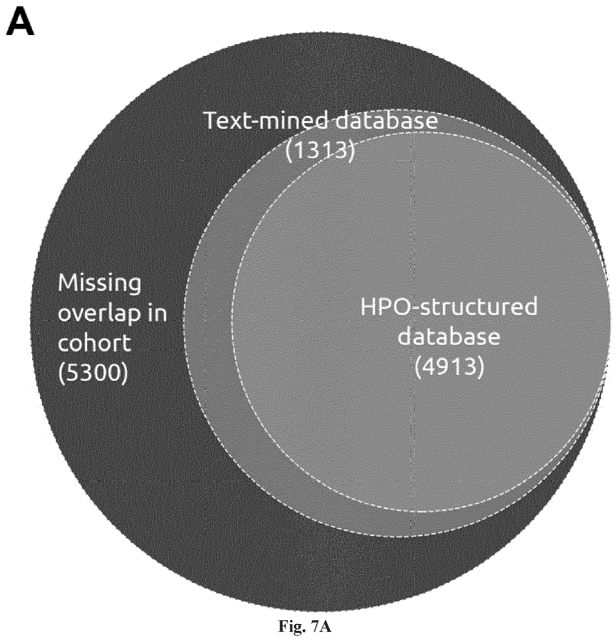

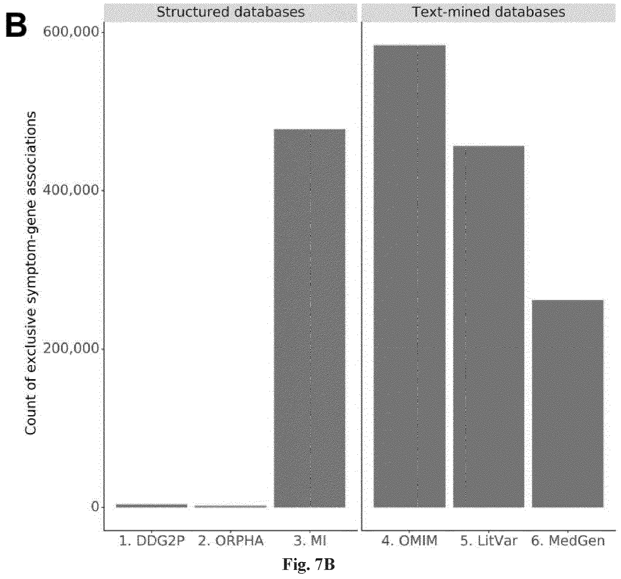

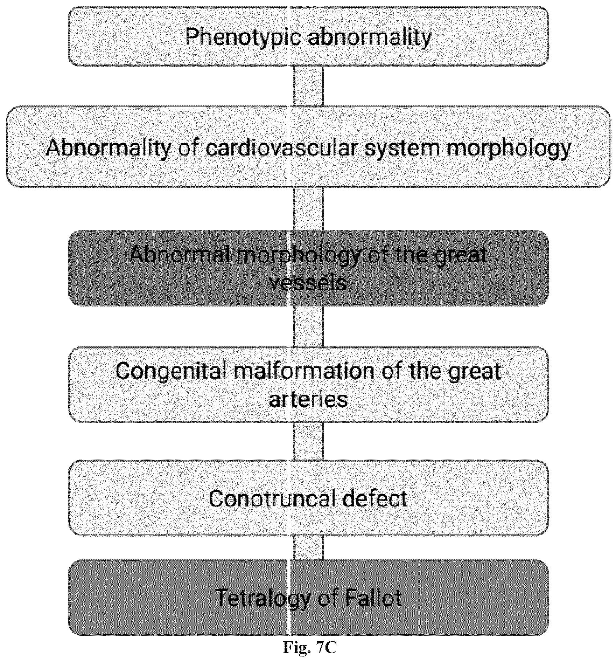

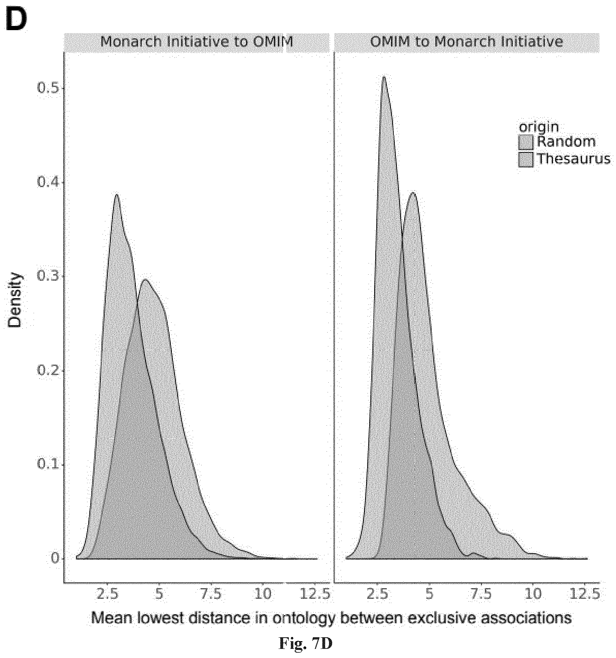

**Figures 7A-7D** are a combination of graphs pertaining to the quantification of the overlap of symptoms-gene associations between the retrospective multicenter cohort of 1,686 patients and the medical literature. **Fig. 7A** shows a Venn diagram of symptom-gene associations observed in cohort overlapped with public HPO-structured databases and text-mined associations in free-text databases. **Fig. 7B** shows a count distribution of symptoms-gene association exclusive to each database. **Fig. 7C** shows an illustration of exclusive symptom-gene associations found in Monarch Initiative database (top plot) and text-mined OMIM database (middle plot), using KMT2D as an example. **Fig. 7D** shows a distribution of the mean lowest distance in the ontology between exclusive terms in the Monarch Initiative database and our text-mined OMIM database, compared to a random choice of HPO terms.
**Figures 8A-8C** are a combination of graphs pertaining to a modeling symptom-symptom interaction in rare diseases using node similarity algorithms on collected symptoms-gene associations. **Fig. 8A** shows a graph visualization of symptom relationships based on the human development architecture of HPO. **Fig. 8B** shows a graph visualization of symptoms relationships with node similarity >80% **Fig. 8C** shows an illustration of symptom relationships with the Kleefstra syndrome clinical report with EHMT1 variant, phenotyped by 12 geneticists.
**Figures 9A-9C** are a combination of graphs pertaining to a modeling symptom-symptom interactions in genetic disease using non-negative matrix factorization. **Fig. 9A** shows a visualization of symptom relationship based on 390 groups of interacting symptoms from medical literature. **Fig. 9B** shows an illustration of group 273 with the main symptom Autoimmunity (HP:0002960). **Fig. 9C** shows a UMAP visualization of cohort's clinical descriptions projected into the group of symptom dimension, colored and annotated by Agglomerative clustering.

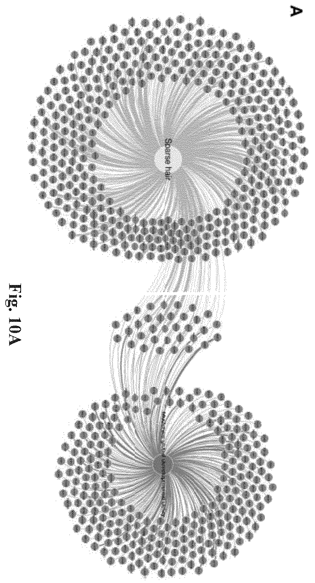

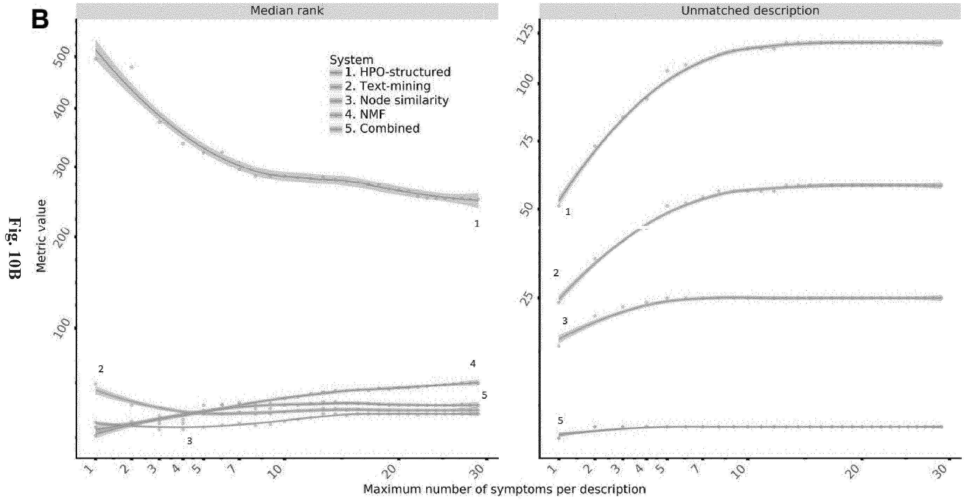

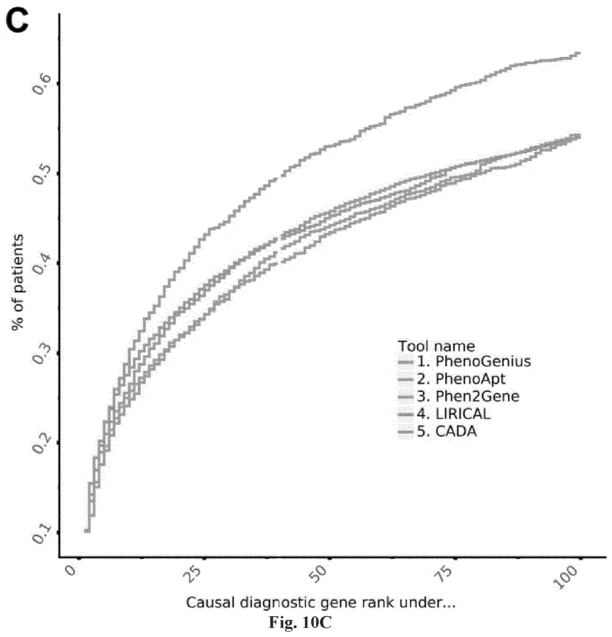

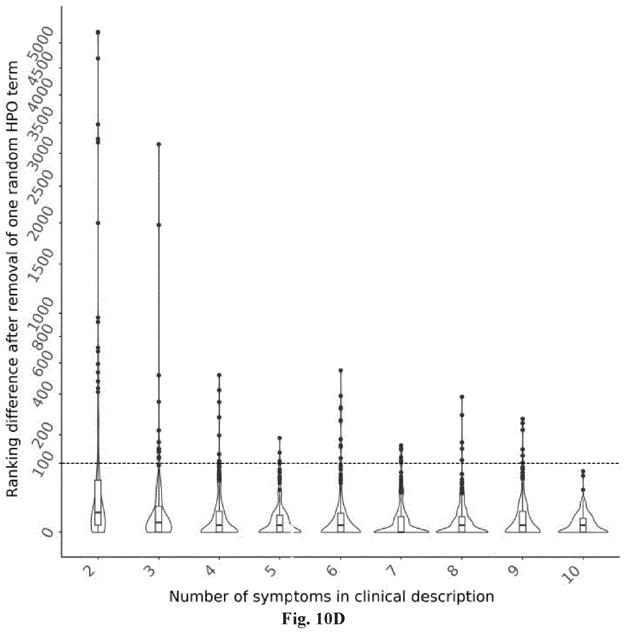

**Figures 10A-10D** are a combination of graphs pertaining to the Modeling of symptom interactions as an efficient system for phenotype matching. **Fig. 10A** shows an illustration of the principle of phenotype matching, looking for the most connected genes to the clinical description containing two symptoms of the Kleefstra syndrome observation with the EHMT1 variant: Sparse hair (HP:0008070, yellow) and Moderate global developmental delay (HP:0011343, purple). **Fig. 10B** shows the representation of performance benchmark metrics of diagnostic gene prioritization ranking (median rank, on the left hand side) and phenotype matching (count of unmatched description, on the right hand side) according to a maximum number of symptoms in clinical descriptions of the cohort. **Fig. 10C** shows a benchmark of a selection of state-of-the-art gene prioritization programs **Fig. 10D** shows ranking differences after removing one symptom according to the number of terms in clinical descriptions.
**Figure 11** represents a treemap chart of the HPO terms in the cohort per main class ontology.
**Figure 12** represent violin plots of the mean depth of HPO terms from root ontology according to each database.
**Figure 13** represents violin plots of pair of symptoms similarity score according to the ontology distance.
**Figure 14** represents a distribution of 1% subsampling of similarity pair score.
**Figure 15** shows a representation of a topic coherence evaluation to determine the optimal number of groups.
**Figure 16** represents a violin plot of symptom-group weight association.
**Figure 17** is a dendrogram of hierarchical clusters of clinical observations obtained using agglomerative clustering on the retrospective cohort using the 16,600 symptoms from HPO.
**Figures 18A-18B** represent UMAP visualizations of clinical description from the retrospective cohort of 1,686 cases using the 16,600 symptoms from HPO.
**Figure 19** represents a UMAP visualization of cohort's clinical descriptions projected using the 16,600 symptoms from HPO, colored and annotated by agglomerative cluster.
**Figure 20** is a dendrogram of hierarchical clusters of clinical observations obtained using agglomerative clustering on cohort projection in 390 groups of interacting symptoms dimension.
**Figure 21** represents a UMAP visualization of cohort's clinical descriptions projected using the 390 groups of interacting symptoms, colored and annotated by agglomerative cluster.
**Figure 22** is a schematic illustration of phenotype matching and gene prioritization system.
**Figure 23** represents a benchmark of a selection of state-of-the-art phenotype-driven gene prioritization per sub-group cohort.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

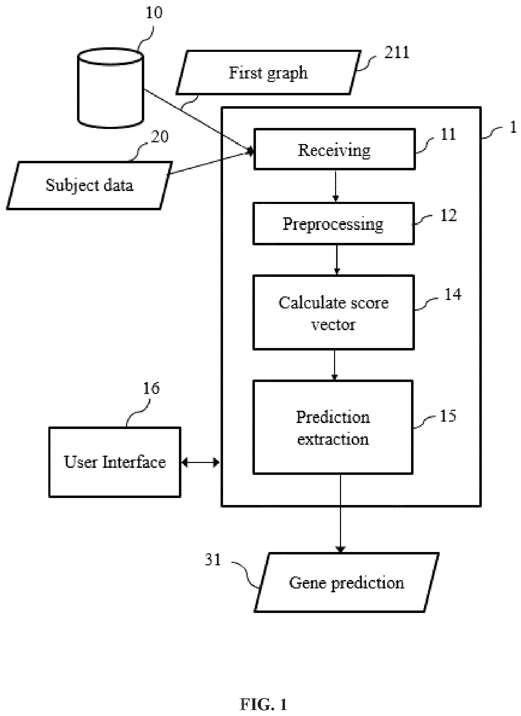

The first aspect of the present disclosure concerning the identification of genes having the highest probability to be associated to symptoms observed for a subject (i.e., by creation of a gene-phenotype matrix) will be described in reference to a particular functional embodiment of a device 1 for the identification of genes having the highest probability to be associated to symptoms observed for a subject and for decision support to standardize phenotyping in genomic analysis of a subject, as illustrated on **Figure 1****.**

The device 1 is adapted to produce such information concerning the gene or the few genes among a set of genes 24, said gene or few genes having the highest probability to be causing the symptoms of the subject from subject data 20 representative of at least one symptom observed for the subject and from information concerning associations between at least one symptom and at least one gene considered to be responsible of the associated at least one symptom.

In one embodiment, the information concerning associations between at least one symptom and at least one gene considered to be responsible of the associated at least one symptom is a gene-phenotype matrix 21, representing an atlas of genotype-phenotype associations. More in details, said gene-phenotype matrix 21 is a matrix *N* × *M.*, wherein *N* is a number of genes and *M* is a number of phenotypes present in a list of associations 22 between at least one phenotype and at least one gene considered to be responsible of said associated at least one phenotype.

The subject data 20, which are representative of the symptoms observable in the subject, may be derived from an electronic heath record associated to the subject.

Though the presently described device 1 is versatile and provided with several functions that can be carried out alternatively or in any cumulative way, other implementations within the scope of the present disclosure include devices having only parts of the present functionalities.

The device 1 is advantageously an apparatus, or a physical part of an apparatus, designed, configured and/or adapted for performing the mentioned functions and produce the mentioned effects or results. In alternative implementations, the device 1 is embodied as a set of apparatus or physical parts of apparatus, whether grouped in a same machine or in different, possibly remote, machines. The device 1 may e.g. have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1.

The device 1 comprises a reception module 11 for receiving for receiving the subject data 20 and the information concerning associations between at least one symptom and at least one gene considered to be responsible of the associated at least one symptom stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

The module 11 may be configured to receive a first graph 211 comprising nodes and weighted links. Each node is representative of one symptom, each symptom being associated to at least one gene. Each link connecting two nodes is representative of a similarity between the two symptoms associated to the two connected nodes. The weight of each link represents a degree of similarity between the two symptoms associated to the two connected nodes. The first graph being previously obtained calculating symptoms pair similarities using information concerning associations between at least one symptom and at least one gene considered to be responsible of said associated at least one symptom. For instance, a node similarity algorithm is used to determine the weight of each link in the first graph. The node similarity algorithm computes pair similarities using the information contained in a gene-phenotype matrix. Advantageously, the first graph can be used to determine a phenotype similar to one given phenotype based on the links and associated weights departing from the given phenotype in the first graph.

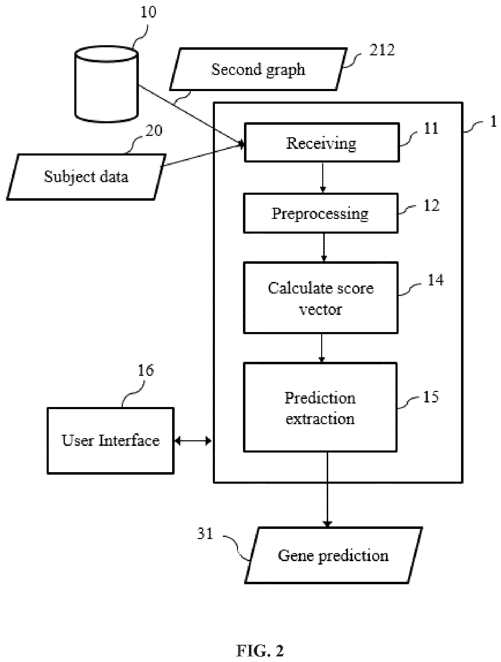

In one alternative embodiment, shown in Figure 2, the reception module 11 may be configured to receive, in alternative to the first graph 211, a second graph 212. The second graph 212 has been previously obtained applying a matrix factorization to a gene-symptom matrix, that can be the first graph 211 and representative of associations between at least one symptom. The second graph 212 comprises nodes and weighted links, wherein each node is representative of one symptom, each symptom being associated to at least one gene. Each node is associated to at least one group of nodes of the second graph 212; and each node is associated to at least one score of specificity, each score of specificity representing the specificity of the symptom associated to the node with respect to the symptoms of the nodes in each group to which is associated said node.

The device 1 further comprises optionally a module 12 for preprocessing the received subject data 20. The module 12 may notably be adapted to standardize the language of the subject data 20 used to report the symptom. Notably, the module 12 is configured to associate each symptom comprised on the subject data 20 to one phenotype, based on a predefined ontology, such as the Human Phenotype Ontology. It has to be noticed that said predefined ontology comprises information concerning all phenotypes represented in the gene-phenotypes matrix.

According to various configurations, the module 12 is adapted to execute only part or all of the above functions, in any possible combination, in any manner suited to the following processing stage.

The device 1 may further comprise a module 14 configured to calculate a score vector representative of the probability that each gene (i.e., gene comprised in the first graph 211 or in the second graph 212) is associated to the symptoms observed for the subject.

The module 14 may be further configured to apply at least one correction factor and/or vector to the score vector. According to one embodiment, the score vector is corrected by a first factor designed to take into account how frequently a variant of each gene is reported as pathogenic in a predefined medical database, such as the database ClinVar (i.e., freely accessible, public archive of reports of the relationships among human variations and phenotypes). Said first factor may be a pathogenic vector constructed so that each of its coefficient *bᵢ* is representative of a frequency at which a variant of each gene i is reported as pathogenic in the predefined medical database. Advantageously, this first correction factor/pathogenic vector allows to put a higher weight to genes more frequently found in diagnosis with respect to genes more rarely found in diagnosis.

More in details, the coefficient of the pathogenic vector for the gene i may be obtained as a ratio having at the numerator the difference between the maximum number of variants expressed by the gene having the largest number of variants (arg max *Var_count*) and the number of variants associated to the gene *i* (*Var_count*(*i*)) and having at the denominator the difference between the maximum number of variants expressed by the gene having the largest number of variants (arg max *Var_count*) and number of variants expressed by the gene having the lower number of variants (arg min *Var_count)* (i.e., (arg max *Var*_count - *Var_count*(*i*))/ (arg max *Var_count* - arg min *Var_count*)).

The score vector may be therefore updated by subtracting to each coefficient *bᵢ* the coefficient of the pathogenic vector obtained for the corresponding gene i. This pathogenic vector may have been previously calculated and stored in the previously cited databased 10. Alternatively, the module 14 may be configured to access the predefined medical database and calculate the pathogenic vector.

The module 14 may be further configured to calculate and apply a second correction factor configured to take into account missing phenotypes associated to gene i that would have not been observed in the subject. This second correction factor may be a correction vector wherein its coefficients are calculated as a function of the coefficients of the rows of the subject specific matrix. More specifically, each coefficient of said correction vector may be calculated as the sum of the coefficients of one row of the subject specific matrix associated to the gene i. This second correction factor is particularly advantageous when only few symptoms (i.e., phenotypes) had been observed in the subject, as short clinical descriptions are usually not accurate enough to select the correct gene.

The score vector may be therefore updated by subtract to each coefficient *bᵢ* of the score vector the coefficient of the correction vector obtained for the gene i. As evident, the first and second correction factors may be applied separately (i.e., the score vector is correct by apply only one correction factor) or jointly (i.e., both correction factors are consecutively applied on the score vector no matter the order). Alternatively, the first and second correction factor, and eventually additional correction factors, may be combined in on global correction factor to apply directly in one step to the score vector.

It may be observed that the operations by the modules 11, 12 13 and 14 are not necessarily successive in time, and may overlap, proceed in parallel or alternate, in any appropriate manner. For example, new image data may be progressively received over time and preprocessed, while the module 13 is dealing with the previously obtained image data. In alternative examples, a batch of image data 21 corresponding to a complete time sequence range may be fully received and preprocessed before it is submitted to the module 13.

The device 1 further comprises a module 15 for extracting prediction 31 from the score vector calculated by the module 14 and for outputting that prediction 31. According to one embodiment, the module 15 is configured to applying a ranking algorithm to the coefficients of the score vector (or corrected score vector if one or more correction factors have been applied). Since the (corrected) score vector comprises the probability for each of the *N* gene of causing the subject disease, the ranking algorithm may provide as information a list wherein the genes are ordered from the one having the highest probability to the one associated to the lower probability. The module 15 may be notably configured to provide as prediction 31 a string of character designating the gene having the higher ranking, (i.e., having the highest probability to be at the origin of the disease of the subject).

The device 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

In one mode of the invention, the device 1 further comprises a module, referred to as matrix module, (not represented) configured to calculate a gene-phenotype matrix, when either the first graph 211 or the second graph 212 is built from such a gene phenotype matrix. In this mode, the receiving module 11 is configured to receive a data set comprising information of associations between genotypes and phenotype. This data set may be a predefined trusted thesaurus, i.e., a list of genotypes associated to the corresponding phenotypes. Among the reference thesaurus it can be cited the Human Phenotype Ontology annotation (HPO). A thesaurus may as well be defined based in the information present in the following datasets: the Online Mendelian Inheritance in Man (OMIM^{®}), MedGen thesaurus, LitVar and the like. The person skilled in the art knows the thesaurus comprising the genotype-phenotype association, or know how to construct a thesaurus, that can be used in the present invention. The at least one thesaurus may be stored in the database 10 or dynamically accessible via a communication network.

The matrix module may be configured to build the gene-phenotype matrix based on one given thesaurus (i.e., list) by implementing the steps described here below. A first step comprises defining a matrix of zeros of dimensions *N* × M, wherein *N* is number of genes and M is the number of phenotypes present in said list comprising associations between phenotypes and genes. In one example wherein the HPO thesaurus is used, the gene-phenotype matrix will have 4531 rows (i.e., number of genes considered) per 15785 columns (i.e., number of phenotypes repertory). A second step comprises attributing a non-zero value to all the coefficients *aᵢⱼ* of the gene-phenotype matrix 21 which represent an association between the gene i and the phenotype j which is comprised on the list. Said non-zero value may be set to be equal to 1.

All phenotypes are linked to each other's and their interconnection may be represented by a hierarchical structure, also called ontology. In one preferred embodiment, said ontology is a direct acyclic graph representative of a standard categorization of the human abnormal phenotypes and of their semantic relationships. In one example, the Human Phenotype Ontology may be used. Advantageously, the gene-phenotype matrix 21 is constructed to correctly reflect the parenting relationships between phenotypes in the gene-phenotypes matrix coefficients.

To do so, a third step is performed on each of the coefficient *aᵢⱼ* still set to zero (i.e., relating to an association of the gene i and the phenotype j absent from the considered list). The value of each of these coefficient *aᵢⱼ* is calculated as function of a distance between the phenotype *j* and its closest phenotype for the same gene *i*, whose association is present in the list. The distance between the phenotype *j* and the closest phenotype is calculated on a direct acyclic graph. Said distance may notably be calculated as the number of nodes present between the phenotype *j* and the closest phenotype related to the same gene i considered. Since taking into account relationship between phenotypes completely disconnected would not provide a correct information, a maximum distance between phenotypes is preferably set, and may be for example equal to a distance of two nodes. Directional barriers may be as well defined on the direct acyclic graph, based on a priori knowledge, so as to avoid associations between certain phenotypes that even having short distance (equal or inferior to the maximum distance) should not be considered as related (ex: HP:0040195 *Decreased head circumference* and HP:0040194 *Increased head circumference).* As disclosed above, the value of each of these coefficients *aᵢⱼ* is calculated as function of the distance, and said function may be linear, exponential, gaussian and the like. In one preferred embodiment, a linear function of decay is used because a linear decay advantageously helps prevents having coefficients values too low. Since the matrix may be too sparse, depending on the list(s) used, it is advantageous to keep significantly high values.

According to one embodiment, the receiving module 11 is configured to receive more than one list and the matrix module is configured to calculate one intermediate gene-phenotype matrices for each respective list comprising associations between at least one phenotype and at least one gene. The intermediate gene-phenotype matrices are calculated for each list as described above. The (final) gene-phenotype matrix is therefore calculated as a combination of the calculated intermediate gene-phenotype matrices. Said combination may be a linear combination and different weights may be associated to the intermediate matrices, for example according to the accurateness of the information comprised in the list used to calculated them. This embodiment advantageously allows to obtain a more robust estimation of the coefficients of the gene-phenotype matrix since lists (i.e., thesaurus) obtained from different sources have been used. Therefore, an eventual incorrect association between phenotype and genotype present in one thesaurus will be mitigated by a more accurate information comprised in one or more others thesaurus.

The matrix module may be further configured to perform a normalization operation on the gene-phenotype matrix after the step of propagation the probabilities according to the phenotypes' relationships represented in the ontology. Advantageously, this embodiment aims at avoiding giving too much weight to the phenotypes that may be too often associated with genes, and on the other hand boosts phenotypes that are less present, and are supposedly more informative. In one embodiment, the normalization is performed by multiplying the gene-phenotype matrix by a normalization a weight vector w, being a column vector of dimension *M*. This operation results in multiplying each column *j* of the gene-phenotype matrix by the weight *w(j)* (i.e., coefficient of the weight vector w).

Each coefficient weight w(*j*) is calculated as a function of the frequency of appearance of the phenotype j with respect to the genes represented in the gene-phenotype matrix 21. Notably, each weight *w(j)* may be calculated as the average of the coefficients of each column j of the gene-phenotype matrix.

According to one embodiment, the device 1 further comprises a panel module (not represented) configured to use the score vector to provide a suggestion of a panel of genes of the subject to be further analyzed. Panel of genes are defined either by user or by database as Australian PanelApp (https://panelapp.agha.umccr.org/).

More in details the present panel module is configured for:
- define a statistically significant symptoms enrichment in gene panel through Fisher test by counting of each HPO term found in matrix for the gene panel group compared to the count for the complete matrix, providing odds ratio and p-value for each HPO-panel association;
- applying a Benjamini Hochberg correction by filtering HPO-panel associations according to a p-value threshold;
- providing a matrix of HPO-panel statistically significant association wherein the rows are associated to the panels, the columns are associated to the HPO terms and the coefficients are the odd ratios of an HPO-panel association;
- prioritization of gene panel available through the score vector (addition of log(Odds Ratio)) of Human Phenotype Ontology enrichments.

According to one embodiment, the device 1 further comprises an informative phenotype module (not represented) configured to suggest at least one informative phenotype according to the at least one phenotype observed in the subject. By informative phenotype, it is to be understood a phenotype yet not observed in the subject but that is potentially interesting to look at more in details, such as for example proposing to perform a cerebral exploration when the only symptom yet reported is a renal cyst.

Said informative phenotype module may be configured to execute the following steps:
- define a non-negative matrix factorization model to summarize the gene-phenotype matrix 21 into a number *T* of columns or topics, wherein *T* is inferior to the number *M* of phenotypes of a gene-phenotype matrix; wherein said model is trained to provide a conversion from a row containing (about 15000 coefficients of values 0 and 1 if the symptom is present) into a row containing about 3200 values;
- transform the information concerning the at least one phenotype into said model in order to suggest topics (i.e., dimension of the latent space where all phenotypes are projected) of HPOs that are the most likely probable.

In some embodiments, the non-negative matrix factorization applied to the gene-symptom matrix comprises a Non-negative Double Singular Value Decomposition (NNDSVD) initialization step.

In some embodiments, said informative phenotype module is configured to compute the second graph 212 based on the T columns or topics defined. The second graph comprises nodes and weighted links. Each node represents one phenotype in the gene-phenotype matrix 21. T groups of nodes are defined in the second graph, wherein each group of nodes corresponds to one among the T columns or topics. Each node in the second graph 212 is associated to at least one score of specificity representing the specificity of the phenotype associated with the node with respect to the phenotypes of the nodes in each group of nodes to which the node is associated. Advantageously, the second graph 212 helps associating a phenotype, associated with a node, to a given group of nodes. In other words, the second graph helps standardizing phenotyping.

Alternatively, the second graph may be computed preparatorily computed by another device executing the steps previously described the for informative phenotype module. The second graph 212 can then be received by the device 1.

Each gene in the gene-phenotype matrix 21 is associated to each group of nodes in the second graph by a gene-group weighting score. The gene-group weighting score of a gene, for a given group of nodes, represents the prevalence of the gene with respect to the group of nodes, that is, to the group of phenotypes corresponding to the group of nodes.

When the informative phenotype module executes the step of transforming the information concerning the at least one phenotype into the non-negative matrix factorization model in order to suggest topics, the informative phenotype module may evaluate the specificity of the received at least one phenotype, using the score of specificity of the node associated the at least one phenotype in the second graph. If the specificity of the at least one phenotype does not satisfy a predefined rule, the informative phenotype module may provide to the user a suggestion of at least one new phenotype, obtained as the phenotype associated to the node in the first graph, computed by the module 17, connected by one link to the node associated to the at least one symptom of the received list of symptoms.

Advantageously, the at least one new phenotype may be received by the module 13 configured to calculate the subject specific matrix, so as to calculate an updated subject specific matrix.

According to one embodiment, the device 1 further comprises a module (not represented) configured to suggestion a metabolic pathway (as defined in the Gene Ontology http://geneontology.org/). Metabolic pathways are groups of genes with the same function in the cell, so it can be considered as a gene panel. According to the similar method of gene-enrichment in clinical symptoms used for panel suggestions, a suggestion of metabolic pathway according to the individual phenotype is provided.

The present invention further relates to a device configured to predict a gene.

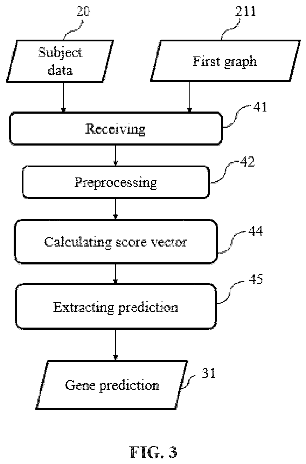

In its automatic actions, the device 1 may for example execute the following process, illustrated on **Figure 3****:**
- receiving the subject data 20, the first graph 211 (step 41),
- preprocessing the subject data 20 to associate each of them to one phenotype j (step 42),
- calculating a score vector representative of the probability that each gene of the subject specific matrix is associated to the symptoms observed for the subject (step 44)
- extracting and providing a prediction 31 based on the score vector (step 45).

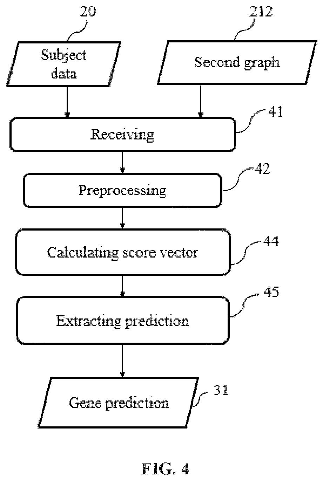

In its automatic actions, the device 1 may for example execute the following process, illustrated on **Figure 4****:**
- receiving the subject data 20 and the second graph 212 (step 51),
- calculating a score vector representative of the probability that each gene of the subject specific matrix is associated to the symptoms observed for the subject (step 55)
- extracting and providing a prediction 31 based on the score vector (step 45).

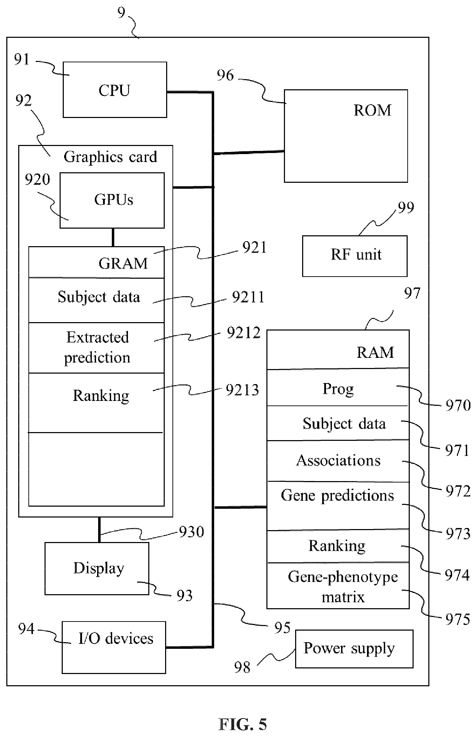

A particular apparatus 9, visible on Figure 5, is embodying the device 1 described above. It corresponds for example to a workstation, a laptop, a tablet, a smartphone, or a head-mounted display (HMD).

That apparatus 9 is suited to gene predictions, to related calculation of gene-phenotype matrices and to decision support to standardize phenotyping in genomic analysis of a subject. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1. In addition, the device 1 may be implemented differently than a standalone software, and an apparatus or set of apparatus comprising only parts of the apparatus 9 may be exploited through an API call or via a cloud interface.

### EXAMPLES

The present invention is further illustrated by the following examples.

An illustration of how the device and the methods previously described can be used practically for the calculation of a symptom-symptom association atlas and its utilization for the identification of one or more gene(s) that has the highest probability to be at the origin of a disease manifested by a subject is given in the following examples.

### Materials and Methods

### Clinical data collection

Anonymized clinical cases from four international cohorts were collected, leading to a total of 1,686 patients with a genetic diagnosis and their clinical description in HPO terms. This cohort is composed of 307 patients gathered from the PhenoGenius consortium from Centre hospitalier universitaire (CHU) Grenoble Alpes, CHU de Dijon, CHU de Montpellier, CHU de Rennes, CHU de Brest and Hospices Civils de Lyon, 140 patients from Seo et al. (1), 298 patients from Trujillano et al. (2), and 941 from Peng et al. (3). We also collected clinical descriptions in HPO format from 12 clinical geneticists from 12 different French hospitals (CHU Lille, CHU Montpellier, CHU Rennes, CHU Rouen, CHU La Réunion, CH Alençon, CHU Poitiers, CHU Limoges, CH Versailles, CHU Toulouse, and CHU Tours). Each physician extracted HPO terms from the same three clinical reports of patients with different diagnostic genes (KMT2D, KMD6A, and C3), one case each from three physicians in French hospitals (CHU Montpellier, CHU Grenoble Alpes, and APHP). Patients or legal guardians provided informed written consent for genetic analyses in a medical setting. Consent from the clinical geneticists was obtained through a survey that also collected their responses.

### Database of medical literature

Databases were downloaded in May 2022. Human Phenotype Ontology was downloaded in OBO format from the Monarch Initiative website (https://hpojax.org/). Clinical databases in HPO format were downloaded from the Monarch Initiative website in the phenotype to genes format, EBI initiative DD2GP's (4) CSV files from https://www.ebi.ac.uk/gene2phenotype/, and Orphanet's XML data from https://www.orpha.net/. Free-text databases were downloaded through API requests for OMIM (https://www.omim.org/), NCBI's MedGen (5) (https://www.ncbi.nlm.nih.gov/medgen/) and NCBI's PubMed abstracts. For the PubMed abstracts, we used the list of all likely pathogenic and pathogenic variants from the ClinVar database (6) to select abstracts of potential interest through LitVar (7) API.

### Text matching algorithm

### Methods

A methodology to extract symptoms-gene associations was developed by the inventors based on Elasticsearch^{®} v5.6 from free-text data in HPO terms and NCBI gene ID format. First, these databases were processed to associate free-text data with the corresponding gene in JSON format. An Elasticsearch query was performed to match every HPO available in each gene-free-text related data and provide a list of HPO-gene associations per database. The number of gene-HPO associations created was limited to the top 100 ranked associated genes for an HPO.

### Mean distance in the ontology between exclusive terms

For each exclusive symptom-gene association from the MI database, an ontology distance for every exclusive symptom-gene association from text-mined OMIM in a common gene was computed and the lowest distance was kept. For each gene, the mean distance in the ontology between exclusive terms of the MI database and our text-mined OMIM database was processed. The same experiment was performed in the opposite direction, from exclusive association in text-mined OMIM database to MI database. To compare the distribution of mean distance against a random distribution, an ontology distance with a randomly selected HPO term instead of an exclusive term from the other database was computed.

### Knowledge data frame structure

A list of symptom-gene associations from each database was stored in a data frame containing 16,600 symptoms in columns and 5,235 genes in rows. Each cell includes the probability of symptom-gene association according to its overlap between databases (consensus score based on a mean, e.g. an association found in half of the databases received a score of 0.5). Databases structured in HPO format (MI, DDG2P, and Orphanet) were considered a unique resource, as DDG2P and Orphanet provided associations mostly overlapping with the MI database and with only 3,492 and 1,849 exclusive associations, respectively.

### Node similarity

The symptoms-gene associations' data frame was transposed into a symptom-symptom association data frame based on symptoms association in the same genes. All existing symptoms to symptoms relationships were injected into a Neo4j^{®} database v4.4.0. The similarity between all pairs of symptoms was processed using the node similarity algorithm (https://neo4j.com/does/graph-data-science/current/algorithms/node-similarity/), and due to technical reasons (RAM limit due to number of combinations), for each symptom, only symptoms with a similarity score > 0.4 were extracted, within a limit of a maximum of 1500 associated symptoms. A similar pair of symptoms was reported if the similarity score was higher than 0.8.

### Collaborative filtering

### Methods

Using sci-kit learn v.0.24.2 Non-Negative Matrix Factorization with Nonnegative Double Singular Value Decomposition initialization, the symptoms-gene associations data frame was transposed into a symptoms-groups of symptoms association data frame based on symptoms association in the same genes. This algorithm provides the numbers of groups requested, the weights of symptom-group associations, and the weight of gene-group associations. For interpretability and illustration, symptoms-group association was filtered to keep only the 10% highest l2-normalized weight of symptoms-group association (>0.04). In phenotype matching evaluation, the complete symptoms-group associations were used.

### Identification of an optimal number of symptoms group

A coherence score metric was applied to processed groups of symptoms to select their optimal number. Using gensim v4.2 implementations of the coherence topic evaluation (https://radimrehurek.com/gensim/models/coherencemodel.html), the range of group numbers with the highest coherence score was sought and also the lowest coefficient of variation using five random state initialization. It was looked for consistency of coherence among different random states to reproduce the same performance with additional data or updates. This is necessary for clinical implementation.

### UMAP and clustering

Sci-kit learn's v0.24.2 agglomerative clustering was implemented (affinity="euclidean" and linkage="ward" parameters), in order to obtain hierarchical clusters. Data were normalized per clinical observations and also per group of symptoms. 75 clusters of clinical descriptions were retained to avoid single observation clusters. Dendrograms were obtained using Scipy v1.8. hierarchy module. UMAP visualization was performed using the umap v0.5.3 module, with the following parameters: neighbours = 3 & minimum distance = 0.9 for 390 groups of symptom dimension and neighbours = 2 & minimum distance = 0.9 for 16,600 symptoms dimension. Those parameters were determined after observing dispersion and clustering of clinical descriptions according to a range of neighbors from (2 to 5) and minimum distance (0.1 to 0.99).

### Graph visualization

Exploration plots were processed using python's plotnine v0.9 package. Graph visualization was obtained using software Gephi v0.9 (https://gephi.org/) with ForceAtlas2 (8) and Neo4j Bloom v2.3. Retina (https://ouestware.gitlab.io/retina/beta/) was used to provide users with a visual browser of graphs.

### Phenotype matching

A phenotype match occurred if at least one symptom in the clinical description was related to the diagnostic gene in the database. The inventors developed a phenotype matching system that matches clinical descriptions with lists of symptoms-gene associations available in the knowledge data frame structure. According to the combination of symptoms from clinical descriptions, the data frame was filtered to contain only selected symptoms or groups of symptoms columns. The sum of the consensus score per row or gene was processed, and genes ranked according to the sum score in descending order. In the case of equal scoring, we applied the worst rank to all equal genes. The evaluation of this phenotype matching using databases in HPO format and text-mined associations used only symptoms declared in the clinical description. The phenotype matching system based on symptoms similarity used declared symptoms and added a virtual symptom containing all highly similar symptoms sharing its weight. The method based on NMF's collaborative filtering projected symptoms into 390 groups of interacting symptoms dimension using a trained model. Each gene is also projected in the 390 groups dimension with different weights. The ranking is calculated based on the normalized Euclidean distance between the 390 group projection of each gene and the patient phenotypes. The gene with the highest distance gets the best ranking.

### Comparisons of phenotype-driven gene prioritization systems

The performance of four different phenotype-driven gene prioritization algorithms was performed: PhenoApt, Phen2Gene, CADA, and LIRICAL (3, 9-11). As each tool provides a different maximum limit for the gene ranking list, the diagnostic performance evaluation was performed based on the top 100 cumulative causal gene ranks to be interpretable. PhenoApt (https://www.phenoapt.org/API) and Phen2Gene (https://phen2gene.wglab.org/api) evaluations were processed using the software's API in May 2022. CADA (https://github.com/Chengyao-Peng/CADA, unique release) and LIRICAL (https://github.com/TheJacksonLaboratory/LIRICAL, v.1.3.4) evaluations were processed using the desktop version via GitHub.

### Statistics

Statistical metrics (Kolmogorov-Smirnov test, Spearman correlation coefficient, Fisher exact test) were obtained using Python's SciPy module v1.8. Benjamini Hochberg correction was obtained using the multipy package v0.16.

### Results

Example 1 and Example 2 aim at showing the heterogeneity in current phenotyping practices and the consequent mismatch between symptom-gene associations found with a given clinical study carried out in a predetermine cohort and symptom-gene associations found in the medical literature.

### Example 1: Phenotyping practices in large cohorts

In this example, the results of a study of current phenotyping practices carried out by the inventors are described to show the heterogeneity in current phenotyping practices.

Table 1 shows the results of a clinical data collection of 1,686 patients in an international cohort. No studies about phenotyping practices in clinical sequencing are known to have been undertaken until now. Through four international studies, 2501 different symptoms in HPO format and 849 different disease-causing genes were collected ⁵⁻⁷.

**Table 1: Clinical data collection of 1686 patients of an international cohort**

| ID | Description | Count of | | | |
|---|---|---|---|---|---|
| | | Patient s | Gene s | Terms Total \| Unique | |
| French multicenter cohort from PhenoGenius consortium | Gathered from CHU Grenoble Alpes, CHU de Dijon, CHU de Montpellier, CHU de Brest, and Hospices Civils de Lyon | 307 | 220 | 3243 | 989 |
| Seo *et al. (1)* | Unselected series of consecutive patients, clinically suspected of carrying a genetic disorder, from non-consanguineous families, who presented at the Medical Genetics Center, Asan Medical Center, Seoul, South Korea, from April 2018 to August 2019. | 140 | 120 | 1135 | 347 |
| Trujillano *et al. (2)* | Consecutive, unrelated patients referred by physicians from 54 countries on different continents have been included in this study. All patients with suspected Mendelian disorders were referred for diagnostic exome sequencing between January 2014 and January 2016. | 298 | 241 | 2411 | 789 |
| Peng *et al. (3)* | Collection of 435 descriptions from German hospitals and 506 ClinVar submissions. | 941 | 528 | 6439 | 1814 |

Table 2 shows the top ten recurring genes in the four groups in the cohort.

**Table 2: Top ten recurring genes in the four groups of the previous cohort**

| Gene name | Count (n=1,686) | Percentage |
|---|---|---|
| *ABCC6* | 22 | 1.29 |
| *ANKRD11* | 21 | 1.23 |
| *ARID1B* | 20 | 1.18 |
| *NSD1* | 18 | 1.06 |
| *BLM* | 16 | 0.94 |
| *FBN1* | 15 | 0.88 |
| *MECP2* | 15 | 0.88 |
| *NF1* | 15 | 0.88 |
| *PTPN11* | 14 | 0.82 |
| *PKD1* | 13 | 0.76 |

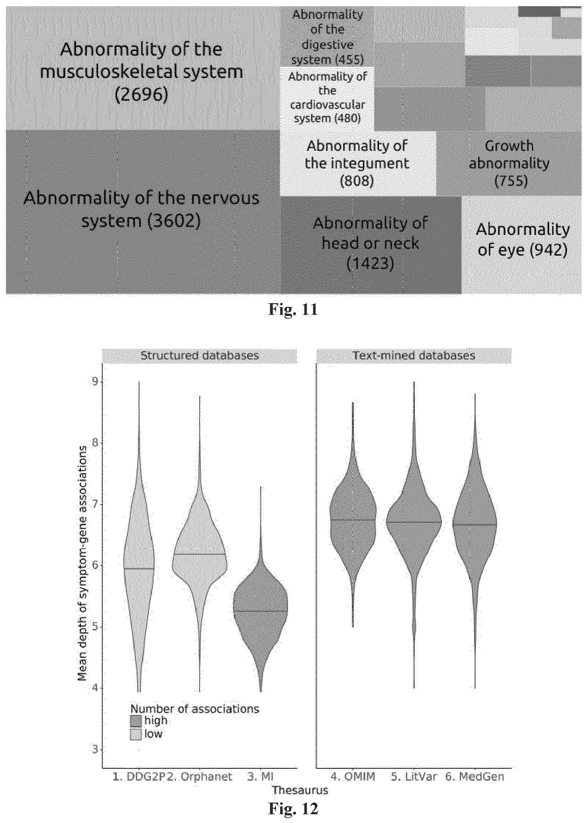

Nearly half of the patients in the multi-center cohort had symptoms belonging to the *Abnormality of the nervous system* (HP:0000707) and *Abnormality of the musculoskeletal system* (HP:0033127) classes, illustrating the current focus on those rare disorders in clinical practice, as can be observed in Figure 11, which is a treemap chart of the HPO terms in the cohort under study per main class ontology. Reflecting the genetic heterogeneity of rare diseases, 538 of 849 genes were declared only once in the cohort. The most frequently mutated gene occurred in less than 2% of cases (as shown in Table 2: ABCC6, n=21).

Table 3 shows the top ten recurring HPO terms in the four groups in the cohort.

**Table 3: Top ten recurring HPO terms in the four groups in the cohort**

| HPO | Description | **Count** (n=13,228) | Percentage |
|---|---|---|---|
| HP:000126 3 | Global developmental delay | 373 | 2.82 |
| HP:000075 0 | Delayed speech and language development | 241 | 1.82 |
| HP:000124 9 | Intellectual disability | 231 | 1.75 |
| HP:000025 2 | Microcephaly | 209 | 1.58 |
| HP:000125 0 | Seizure | 205 | 1.55 |
| HP:000125 2 | Hypotonia | 170 | 1.29 |
| HP:000432 2 | Short stature | 168 | 1.27 |
| HP:000127 0 | Motor delay | 126 | 0.95 |
| HP:000162 2 | Premature birth | 108 | 0.82 |
| HP:000048 6 | Strabismus | 102 | 0.77 |

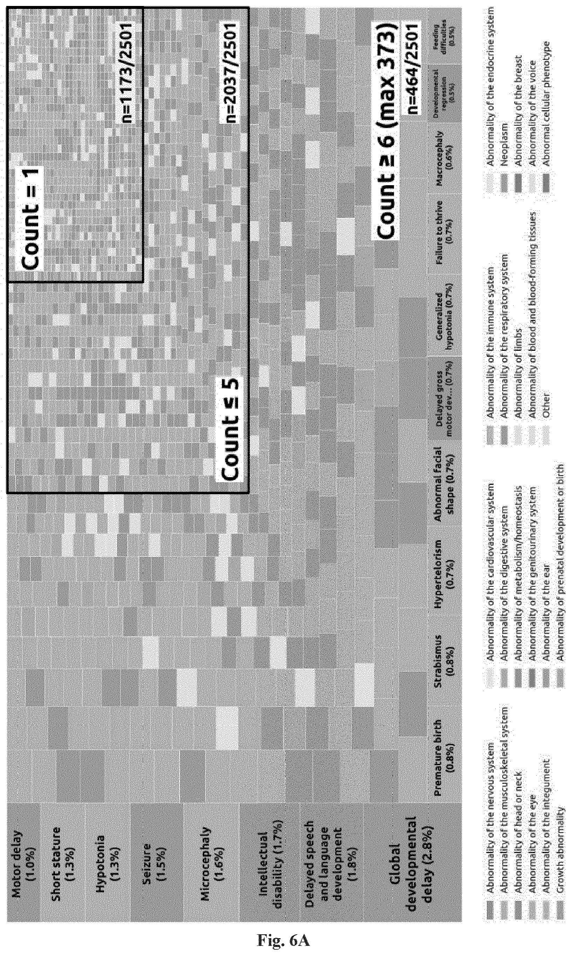

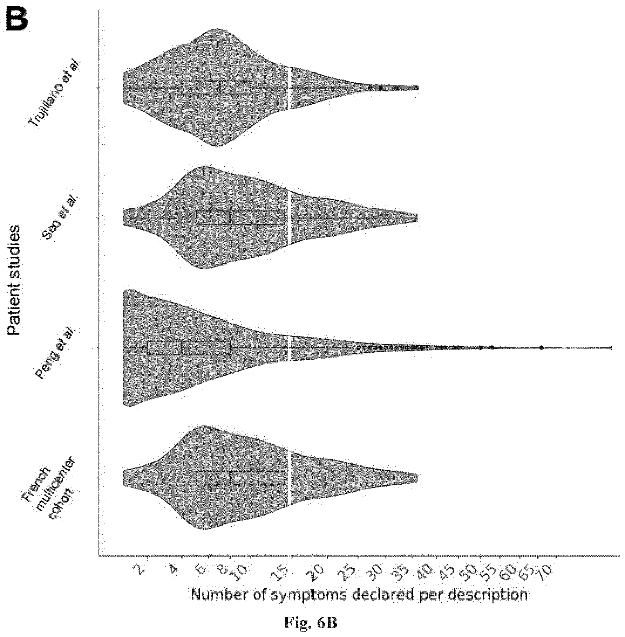

A heterogeneity in HPO selection terms was observed, as 47% of terms were used only once (as shown on Figure 6A, and in Table 3). The median number of HPO terms per physicians' clinical description varied across observations, ranging from three (Peng et al. ⁷) to seven (PhenoGenius consortium, Seo et al. and Trujillano et al. ^{5,6}) as observed on Figure 6B, which is a violin plot of HPO term counts per clinical description for each among the four subgroups of the cohort under study. The heterogeneity of physicians'clinical descriptions was also observed for patients with identical genetic diagnoses. For genes involved in diagnosis of more than ten patients, 67 % of symptoms were declared in only one clinical description.

Table 4 is a description of clinical geneticist profiles in the prospective phenotyping experiment.

**Table 4: Description of clinical geneticist profiles in the prospective phenotyping experiment**

| ID | Profile | Self-estimate expertise in phenotyping using HPO format (from 1 to 10) |
|---|---|---|
| 1 | Clinician | 7 |
| 2 | Clinician | 1 |
| 3 | Clinician | 5 |
| 4 | Resident in medical genetics | 9 |
| 5 | Clinician | 5 |
| 6 | Clinician | 1 |
| 7 | Clinician and Laboratory Specialist | 1 |
| 8 | Clinician | 6 |
| 9 | Clinician and Laboratory Specialist | 5 |
| 10 | Resident in medical genetics | 6 |
| 11 | Clinician | 3 |
| 12 | Resident in medical genetics | 1 |

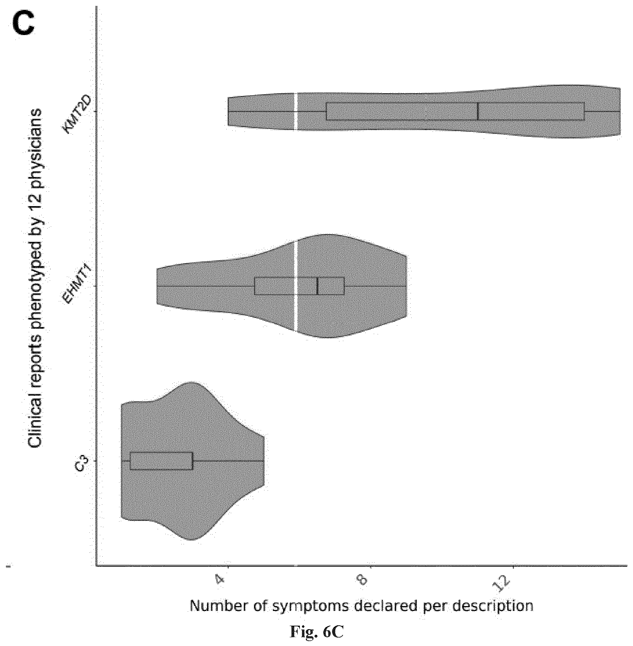

To exclude the possibility that the observed heterogeneity was due to variability in clinical examinations, it was next investigated by the inventors whether heterogeneity in clinical descriptions was reported if physicians phenotyped the same clinical observations. A prospective experiment was settled, where 12 clinical geneticists with various levels of expertise, as presented in Table 4, were asked to phenotype three independent clinical reports associated with genetic test prescription, i.e. to convert free text to phenotypes in HPO format. Heterogeneity in terms of the number and diversity of symptoms declared per clinical observation were observed, as shown on the violin plots of Figure 6C. Those are the violin plots of HPO terms counts per clinical description for each clinical report phenotyped by the 12 clinical geneticists. For instance, two to nine symptoms were declared in clinical descriptions of the Kleefstra syndrome observation with the EHMT1 pathogenic variant. A total of 29 different terms were provided; 17 of these terms were used by two or more physicians, and none of the terms were mentioned by all 12 physicians.

### Example 2: Quantifying the overlap of symptoms-gene associations between the retrospective cohort and the medical literature

In this example, the results of a study aiming at quantifying how symptoms-gene associations obtained from the retrospective cohort of Example 1 and symptoms-gene associations obtained from the medical literature overlap.

To assess if the clinical descriptions of the cohort used in Example 1 matched available knowledge in the medical literature, a mapping between the cohort's 11,526 unique symptom-gene associations and the 734,931 associations available in HPO-structured databases (Orphanet, DDG2P ⁹, and the Monarch Initiative or MI ³) was performed. From these databases, only 4,913 associations (43%) matched, meaning that 57% were missing, as shown on Figure 7A, which is a Venn diagram of symptom-gene associations observed in cohort overlapped with public-HPO-structured databased and text-mined associations in free-text databases.

As the clinical descriptions of genetic diseases in medical literature are mainly available in free-text format, a text-mining algorithm was developed based on Elasticsearch to extract symptom-gene associations from free-text data in HPO format. Applied to OMIM ¹⁰, MedGen ¹¹, and abstracts from PubMed, this text-mining algorithm identified an additional 1,049,522 symptom-gene associations. This approach resulted in a 3.2-fold increase in HPO-structured database associations, as shown on Figure 7B, which represents the count distribution of symptoms-gene associations exclusive to each database.

The text-mining algorithm provided symptom-gene associations where symptoms were significantly deeper in the ontology, i.e. in text-mined databases, compared to the HPO-structured databases. The respective median depths were 6.7 and 5.2, with Kolmogorov-Smirnov test p-value < 10⁻²¹⁵, as illustrated on the violin plots of Figure 12 representing the mean depth of HPO terms from root ontology according to each database. This underlines the complementarity of these approaches, as illustrated in Figure 7C where KMT2D was associated with *Abnormal morphology of the great vessels* (HP:0030962) in the Monarch Initiative database and *Tetralogy of Fallot* (HP:0001636) in the OMIM database. Reflecting the variability across individuals in selecting an HPO term to summarize a clinical observation, 76% of associations were exclusive to one database. The inventors hypothesized that text-mined symptom-gene associations in the literature were related to associations available in HPO-structured databases. This hypothesis embodies the fuzzy phenotyping concept, providing human-determined alternative wordings of the same information.

To evaluate this hypothesis, for each gene, a comparison was performed between the average distances in the ontology of exclusive symptom-gene associations to the MI database and to the text-mined OMIM database, respectively the largest database of each type (structured or text-mined), as illustrated on Figure 7D, which represents the distribution of the mean lowest distance in the ontology between exclusive terms in the MI database (graph on the left) and the text-mined OMIM database (graph on the right), compared to a random choice of HPO terms (curve on the right on each graph). Compared with a random choice of an HPO term, the average distance of the exclusive symptom-gene associations was significantly lower, suggesting these associations are related. The Kolmogorov-Smirnov test p-value was less than 10⁻²¹⁵ in the results presented on Figure 7D.

Although in this exercise the number of symptom-gene associations increased from 734,931 (MI, DDG2P, Orphanet database) to 1,784,453 (with associations found with the text mining algorithm), a match with the cohort's symptom-gene associations was only available for 6,226 of 11,526 (57%) associations, meaning that 43% of matches were still missing, as shown on the Venn diagram of Figure 7A.

### Example 3: From symptom-gene to symptom-symptom associations modeling

In this example, il will be shown how the symptom-symptom associations modeling presented in the present application improve the coverage of symptom-gene associations in the cohort. In particular, it will be shown how modeling associations between symptoms of the same genetic disorder improved matches. The symptom-symptom associations modeling corresponds to the first graph 211 previously described.

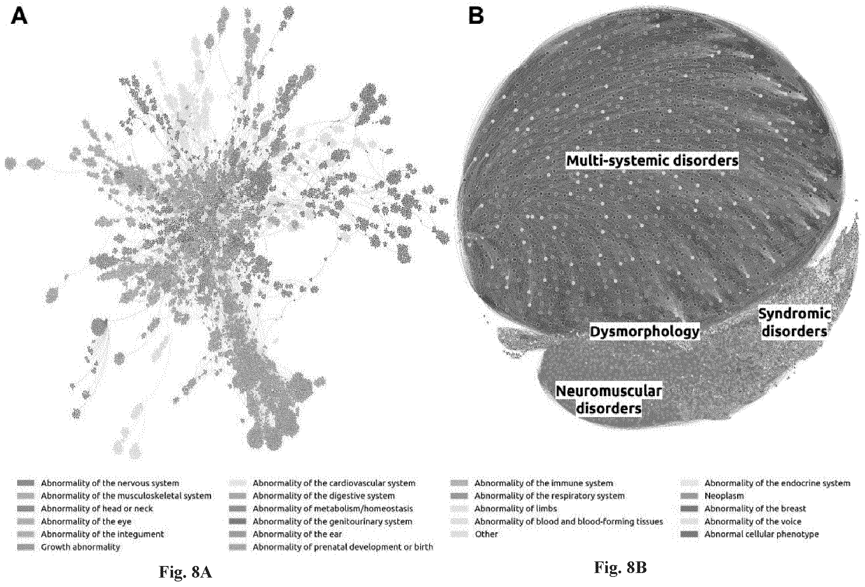

As the Human Phenotype Ontology is ordered according to human development, it may not represent the interaction of symptoms in disease, as shown on Figure 8A where the different gray levels represent different symptoms. An alternative approach was thus explored to measure symptom-symptom associations in genetic diseases. More specifically, a node similarity algorithm based on a knowledge graph that stored the symptom-gene associations collected from the literature was implemented.

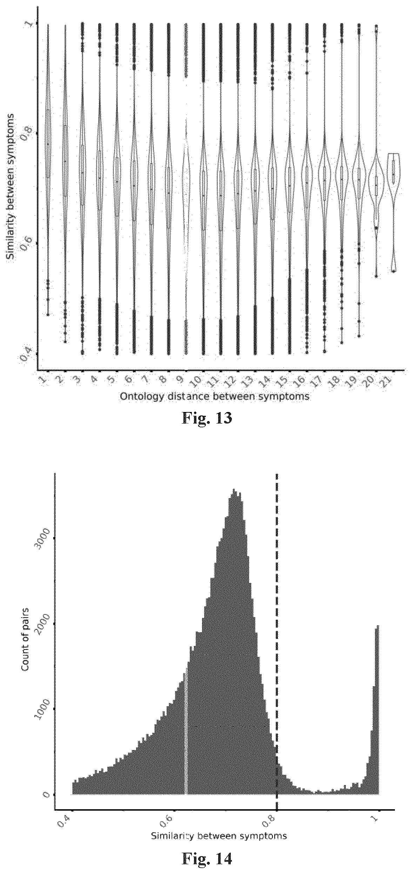

This implementation resulted in the finding of a high correlation between symptom-symptom similarity pair scores and their frequency of co-occurrence in clinical observations (Spearman correlation coefficient: 0.99). No correlation was observed between symptom-symptom similarity pair scores and the distance between symptoms in the HPO (Spearman correlation coefficient: -0,02, Figure 13), reflecting that symptom-symptom associations cannot be solely derived from the ontology architecture.

According to similarity score distributions, it was posited that similarities above 80% were potential synonyms or highly similar symptoms in diseases, threshold schematically represented by the vertical dashed line on Figure 14. This resulted in the selection of 565,943 pairs of highly similar symptoms, corresponding to the 10% highest symptom-symptom association scores (Figure 8B). A total of 26% of these pairs were observed for symptoms in the same ontology class (145,611 of 565,943), mostly from the *Abnormality of the musculoskeletal system* (HP:0033127) class (51%, 73,817 of 145,611). Inter-classes pairs of symptoms represented 74% of highly similar symptoms, where the most recurrent pair was *Abnormality of metabolism*/*homeostasis* (HP:0001939) with *Abnormality of the nervous system* (HP:0000707) (8%, 35,476 of 420,332).

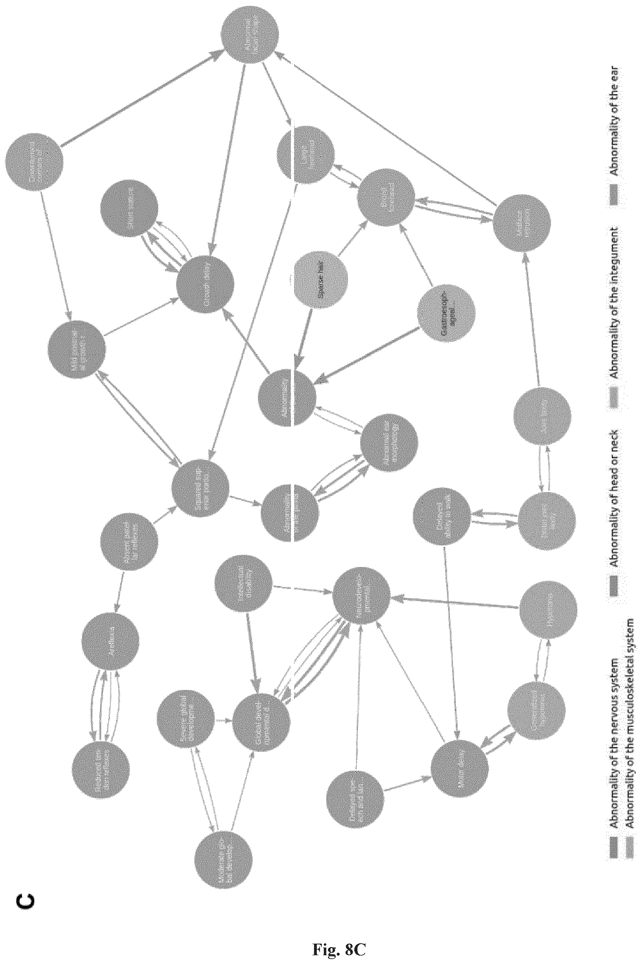

Those similarities are illustrated in Figure 8C, using the symptom *Hypotonia* (HP:0001290) reported by six of the 12 practitioners in our exercise on the Kleefstra syndrome with the *EHMT1* pathogenic variant. Dashed arrows link the closest symptom in HPO ontology and plain arrows the symptom with the highest node similarity among declared symptoms. In the symptom-symptom association graph, the closest term to Hypotonia is *Neurodevelopmental delay* (HP:0012758), with a symptom-symptom similarity pair score measuring 86%. In the HPO, these symptoms are separated by ten nodes and belong to two different main classes: *Abnormality of the musculoskeletal system* (HP:0033127) and *Abnormality of the nervous system* (HP:0000707) respectively. Figure 8C is an example of a portion of the first graph previously presented.

It was further investigated to what extent considering two highly similar symptoms as synonyms improved the coverage of symptoms-gene associations. Among the cohort's 11,526 unique symptom-gene associations, only 6,226 associations were found in HPO-structured and text-mined databases, but this number rises to 8,350 when accounting for similarities. Considering synonymy provided additional 1,506,469 symptom-gene associations to the previous 1,784,453 associations from MI, DDG2P, Orphanet, and text-mined databases.

It can thus be concluded that modeling associations between symptoms revealed a majority of inter-HPO classes included similar symptoms, highlighting the missing aspect of symptom relationships in the HP ontology. Enhancing symptoms with their highly similar pairs improved coverage of symptom-gene associations in the cohort. However, 27% of associations were still missing.

It will be shown in the next example (example 4) that the latter missing can be improved by the groups of symptoms modeling described in the present disclosure.

### Example 4: From symptom-gene associations to groups of symptoms modeling

In Example 3, symptom-symptom associations were evaluated independently when constructing synonyms based on node similarity. To gain better coverage of symptom-gene associations, a more elaborate collaborative filtering approach based on non-negative matrix factorization (NMF) was followed in the present example. More specifically, Example 4 illustrates the computation of a second graph as previously described 212.

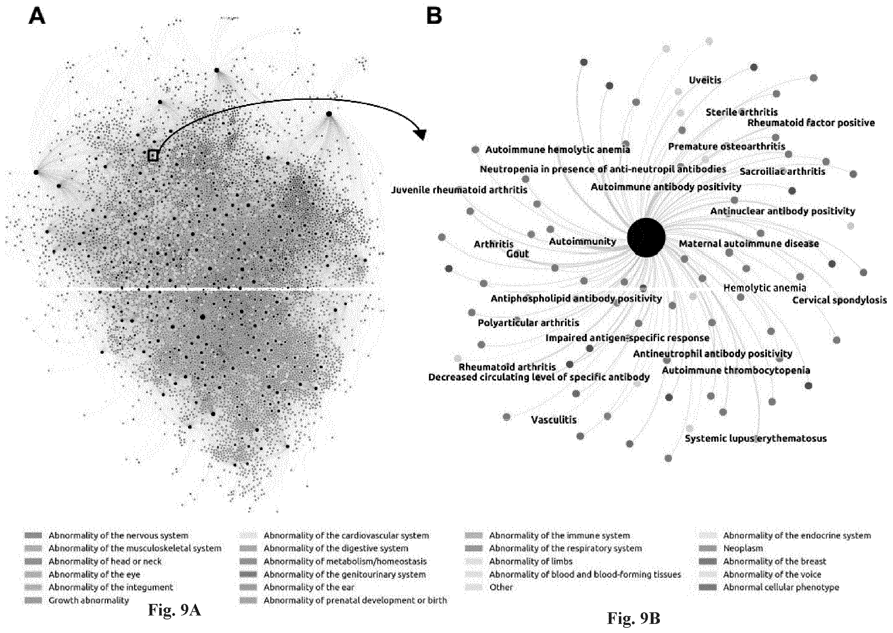

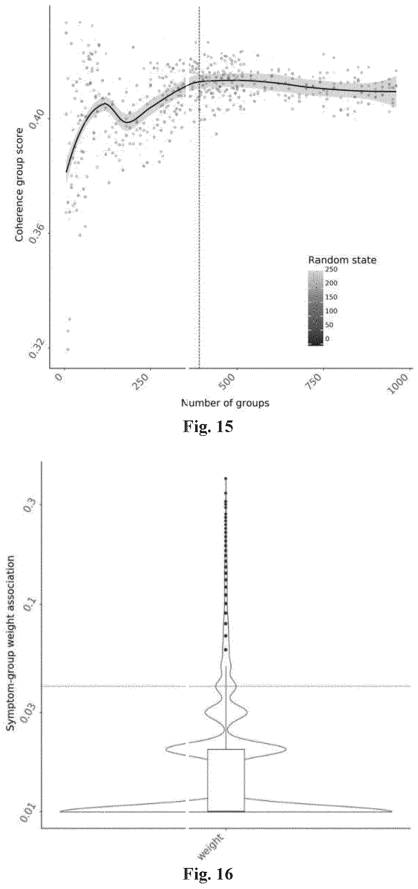

Using the topic coherence measure, it was determined that the 16,660 HPO terms could optimally be reduced to 390 groups of interacting symptoms or phenotypic patterns (Figure 15, where each circle corresponds to one among iterative training experiments according to random state initialization). Each symptom was positioned in the graph with group weights determined by the algorithm, as illustrated on Figures 9A-9B. Figure 9A represents a visualization of symptom relationship based on the 390 groups of interacting symptoms, where the bold black square represents a particular group (group 273) Figure 9B illustrates more in details group 273 with the main symptom Autoimmunity (HP:0002960). On Figures 9A and 9B, the line thickness is proportional to the weights of symptoms in the group. Groups are in black. For readability, only the top 10% of symptom-group associations are displayed. Each gene was associated in a median of 36 groups and a group with a median of 501 genes. To compare the recall of the NMF and the node similarity model, only the top 10% of 390 symptom-groups weights was kept, as shown on Figure 16, which is a violin plot of symptom-group weight association. On Figure 16, the dashed horizontal line corresponds to the top 10% of symptom-group associations normalized weight threshold (larger than 0.04) Overall, in this selection, there were 43,308 symptom-group associations leading to 5,971,755 pairs of symptoms.

It was further investigated to what extent the coverage of symptoms-gene associations was improved by considering that two symptoms belonging to the same group were synonyms. Using these pairs of symptom associations enhanced the coverage of symptom-gene associations to 11,340 of the 11,526 associations from the cohort, leaving less than 2% of matches missing. This new manner of detecting associations resulted in the addition of 2,163,663 NMF-based symptom-gene associations to the previous 1,784,453 associations obtained from MI, DD2P, Orphanet, and text-mined databases. NMF-based symptom-gene associations overlapped with 99% of similarity-based associations (1,497,601 of 1,506,469).

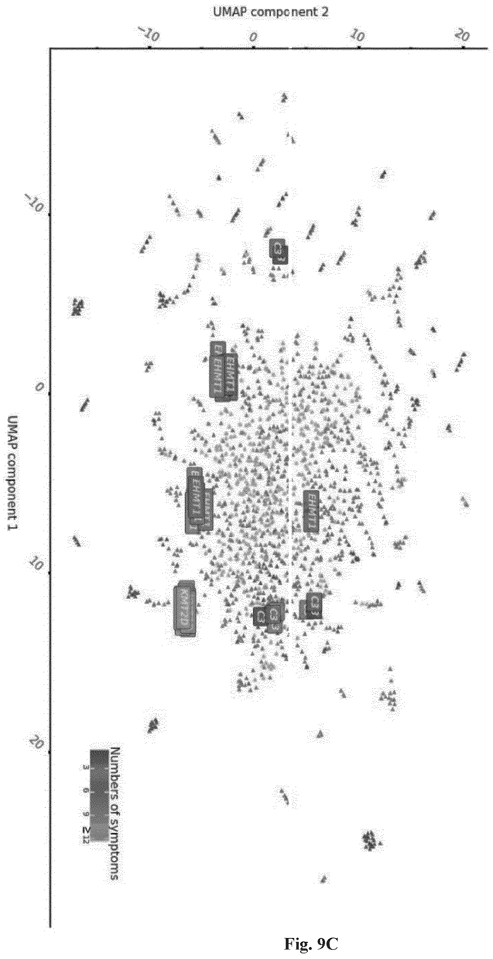

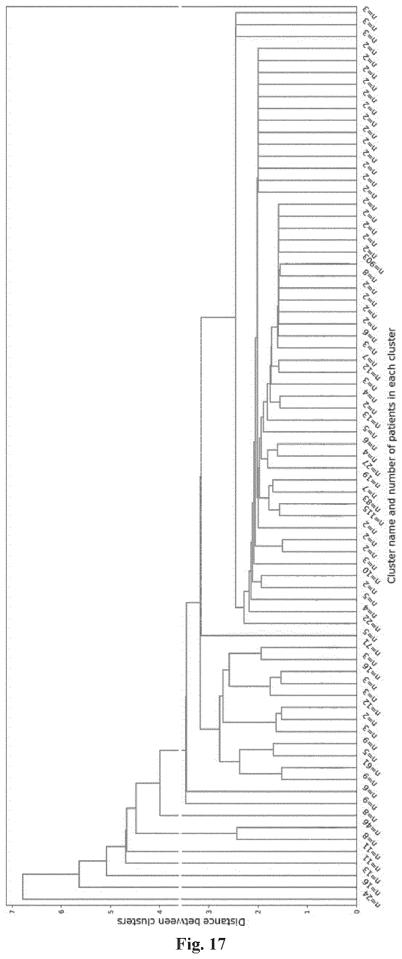

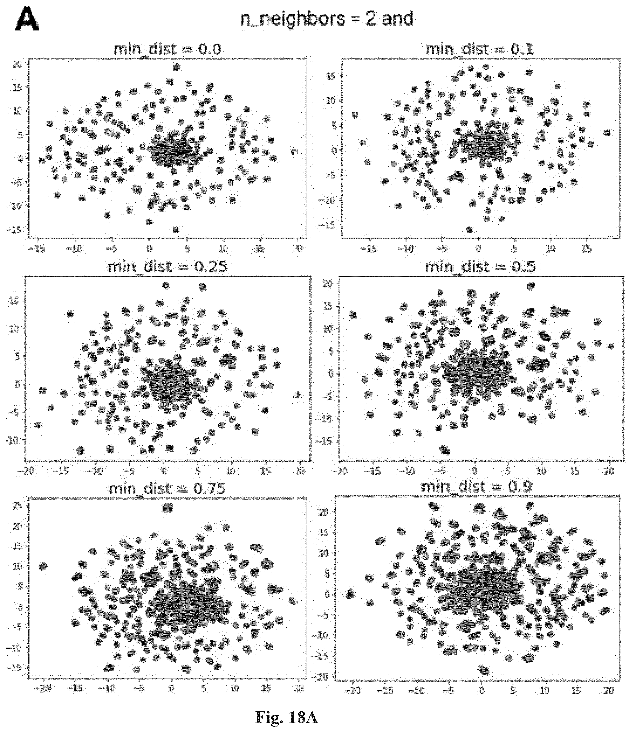

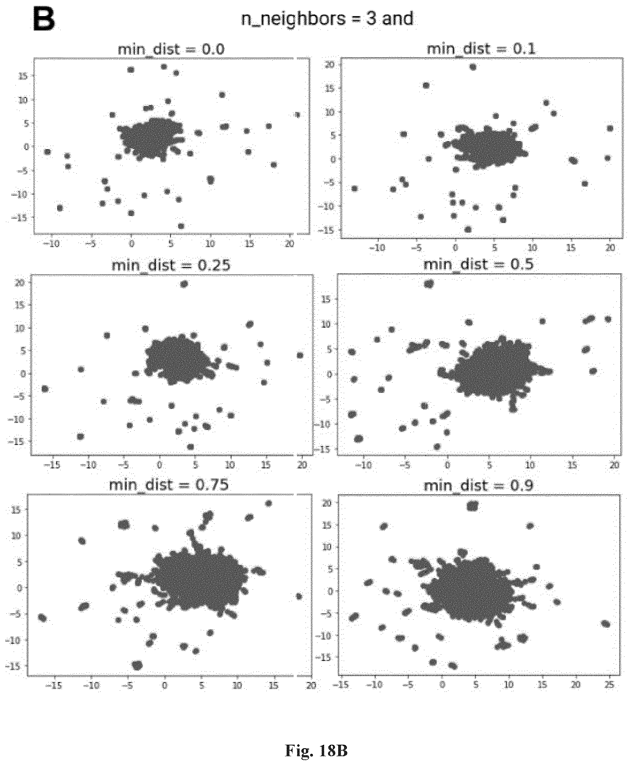

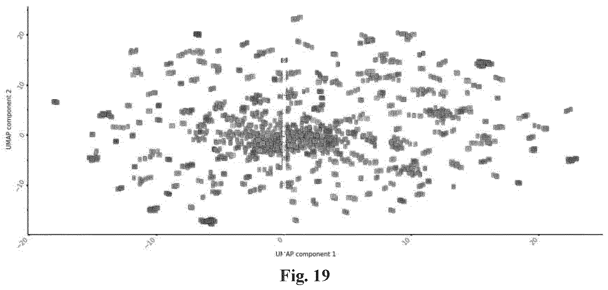

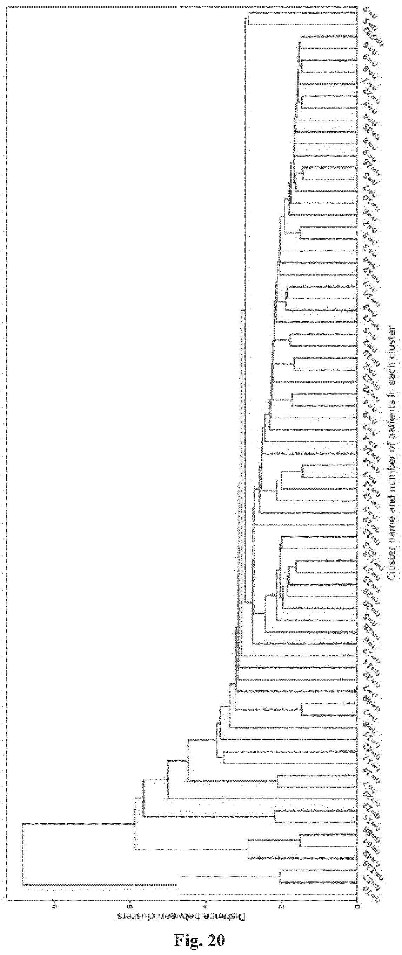

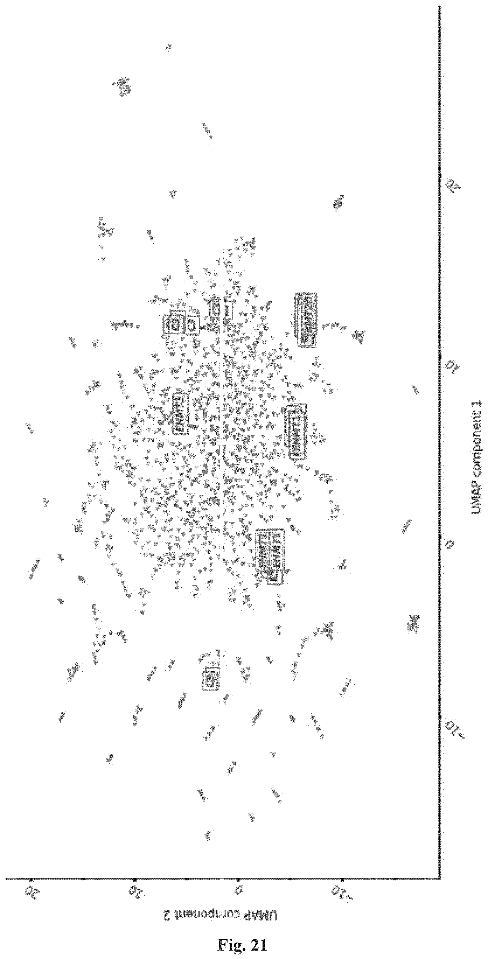

To evaluate if these 390 phenotypic patterns represented the clinical spectrum of genetic diseases, we projected the cohort into the groups of symptoms dimension and performed a UMAP visualization (Uniform Manifold Approximation and Projection) ¹⁴. Agglomerative clustering was applied to the cohort and a comparison of the clustering patient performance using this projection and the 16,600 HPO dimension was performed. Using the initial list of 16,600 symptoms, 152 patients were found, referring to Figures 17 to 19, in 14 clusters significantly enriched in symptoms (Fisher exact test with p-value < 0.05 with Benjamini Hochberg correction). Applying the projection in groups of symptoms, 1,136 patients were found in 51 clusters significantly enriched in groups of symptoms, as shown on Figure 20 and Figure 21. The projection was applied to the three clinical reports phenotyped by the 12 physicians in our experiment presented in Table 4, to evaluate if this projection can standardize clinical descriptions. A high coherence of the projection method even with symptom heterogeneity was demonstrated when sufficient numbers of HPO terms were given (KMT2D report), referring to Figure 9C, which represents the UMAP visualization of cohort's clinical descriptions projected into the group of symptom dimension, colored and annotated by Agglomerative clustering. When fewer than 5 terms were provided, clinical description projections still grouped patients but with lower homogeneity (*EHMT1, C3).*

Thus, those results show that the delineation of 390-groups of interacting symptoms, by following the more elaborate collaborative filtering approach based on non-negative matrix factorization (NMF), enabled an increase in coverage of the available knowledge on genetic disorders and provided a way of building on HP ontology to standardize clinical descriptions.

### Example 5: Symptom interaction models as an efficient and robust system for phenotype matching

In this example, the use of the previously described symptom interaction modeling, and illustrated in Example 3 and Example 4 to develop a phenotype matching system is presented.

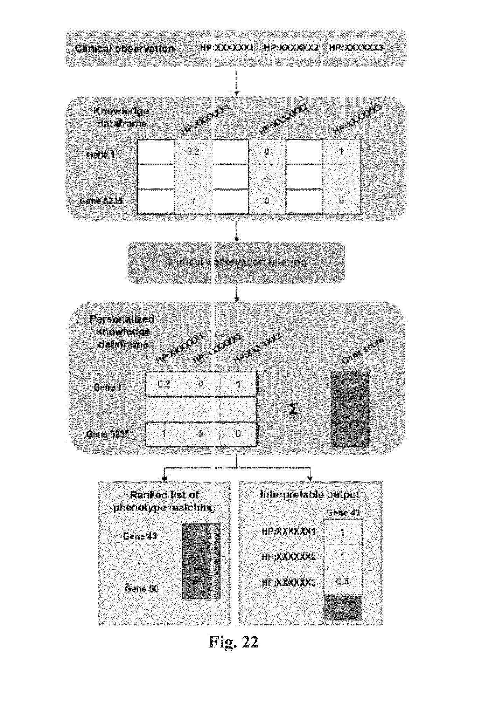

To evaluate the clinical relevance of symptom interaction models, both a phenotype matching experiment and a diagnostic gene ranking experiment were designed and carried out by the inventors. A phenotype match is defined when at least one symptom in the clinical description was related to the diagnostic gene, as schematically illustrated on Figure 10A. Figure 10 A gives an illustration of the principle of phenotype matching, looking for the most connected genes to the clinical description containing two symptoms of the Kleefstra syndrome observation with the *EHMT1* variant: *Sparse hair* (HP:0008070, yellow) and *Moderate global developmental delay* (HP:0011343, purple). Figure 10A thus shows two examples of group of nodes of a typical second graph as previously presented. Line thickness is proportional to the probability score of symptom-gene associations available with joint HPO-structured and text-mined databases. According to the count of matches per gene, a personalized ranked list of genes was provided, as illustrated on Figure 22. These experiments were performed on the clinical observations of 1,686 patients.

Using the HPO-structured databases (MI, DDG2P, Orphanet), a phenotype match was obtained for 1,566 clinical observations with a median diagnostic gene rank of 251. Applying text-mined associations led to a match for 1,628 clinical observations with a median rank of 40. Figure 10B illustrates two performance benchmark metrics of diagnostic gene prioritization ranking (median rank, on the left hand side) and phenotype matching (count of unmatched description, on the right side) according to a maximum number of symptoms in clinical descriptions of the cohort. The best performance in median diagnostic rank was provided by node similarity symptoms association (curve #3 on Figure 10B) (median rank 37, compared to 58 with non-negative matrix factorization -NMF), but NMF (curve #4) was able to get a more exhaustive coverage of clinical observations (1682, compared to 1663 with node similarity). This coverage gap was exclusively observed where the clinical descriptions contained five terms or less (four unmatched descriptions, compared to 25 with node similarity). As each symptom interaction model provides a different level of inductive reasoning, one among the two models was conditionally applied according to the number of symptoms in the clinical description. The combined system, which the Applicant called PhenoGenius, provided the best performance (median rank 41) and reached a nearly full phenotype match of diagnostic genes (99.8%, 1682/1686) for all clinical descriptions.

To illustrate this phenotype-matching system, we considered a clinical description containing two symptoms of the Kleefstra syndrome observation with the *EHMT1* pathogenic variant: *Sparse hair* (HP:0008070) and *Moderate global developmental delay* (HP:0011343). There is no match between these terms and *EHMT1* in HPO-structured databases. No match is identified from text-mined symptom-gene associations either. The symptom interaction modeling according to the present disclosure achieves phenotype matches, ranking 1244 out of 5235 (top 25% of genes) with the similarity model and 851 out of 5235 (top 17% of genes) with the NMF model and PhenoGenius combined system.

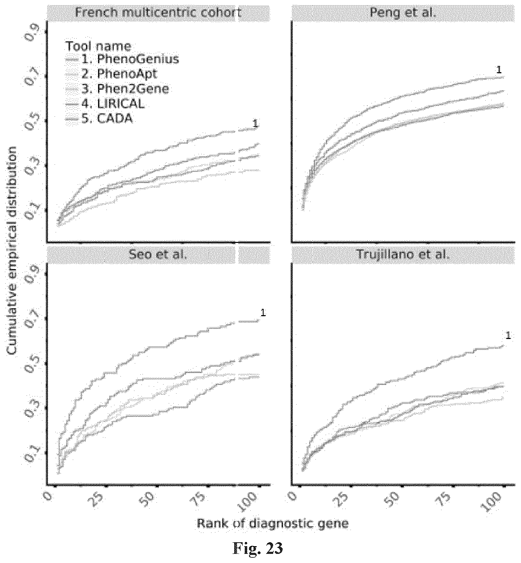

The combined system (PhenoGenius) was compared to four recently published algorithms for phenotype-driven gene prioritization: PhenoApt, Phen2Gene, CADA, and LIRICAL ^{7,15-17}. Despite using different prioritization methodologies, these four programs demonstrated similar performances in phenotype matching, as shown on Figure 10C, where the top-most curve corresponds to the combined system, while the closely-positioned curves below represents the four algorithms under comparison. Using symptom interaction modeling, the PhenoGenius combined system (median rank 41) increased the median diagnostic gene rank by 42% compared to the best competitor, Phen2Gene (median rank 71, 73 to 80 for other methods). This improvement was replicated across each study subgroup in the cohort, highlighting the clinical relevance of symptom interactions in genetic disease models. Figure 23 shows the results of this benchmark, where on each graph, the top curve corresponds to the result obtained with the PhenoGenius combined system.

A further study was carried out aiming at assessing the robustness of gene prioritization. For each symptom from clinical descriptions, two terms or more were randomly removed with and the consequence on the disease-causing gene ranking for descriptions in the top-ranked half of the cohort (rank 41 or lower) was measured. Overall, 701 clinical descriptions led to 6,331 symptom removal experiments. In most cases, phenotype matching remained robust with symptom removal, as illustrated on Figure 10D. Disease-causing gene ranking was identical in 35% of cases (2,274 of 6,331) and the median of absolute differences between ranks was only one. However, nine extreme drops in the ranking (> 1000) were observed with clinical descriptions with three or fewer terms, including two complete loss of phenotype matches for descriptions with two symptoms. For clinical descriptions with four or more terms, no extreme drop in gene rankings was found.

### References of the Material and Methods Section

1. G. H. Seo, T. Kim, I. H. Choi, J.-Y. Park, J. Lee, S. Kim, D.-G. Won, A. Oh, Y. Lee, J. Choi, H. Lee, H. G. Kang, H. Y. Cho, M. H. Cho, Y. J. Kim, Y. H. Yoon, B.-L. Eun, R. J. Desnick, C. Keum, B. H. Lee, Diagnostic yield and clinical utility of whole exome sequencing using an automated variant prioritization system, EVIDENCE. Clin. Genet. 98, 562-570 (2020).
2. D. Trujillano, A. M. Bertoli-Avella, K. Kumar Kandaswamy, M. E. Weiss, J. Köster, A. Marais, O. Paknia, R. Schröder, J. M. Garcia-Aznar, M. Werber, O. Brandau, M. Calvo Del Castillo, C. Baldi, K. Wessel, S. Kishore, N. Nahavandi, W. Eyaid, M. T. Al Rifai, A. Al-Rumayyan, W. Al-Twaijri, A. Alothaim, A. Alhashem, N. Al-Sannaa, M. Al-Balwi, M. Alfadhel, A. Rolfs, R. Abou Jamra, Clinical exome sequencing: results from 2819 samples reflecting 1000 families. Eur. J. Hum. Genet. 25, 176-182 (2017).
3. C. Peng, S. Dieck, A. Schmid, A. Ahmad, A. Knaus, M. Wenzel, L. Mehnert, B. Zirn, T. Haack, S. Ossowski, M. Wagner, T. Brunet, N. Ehmke, M. Danyel, S. Rosnev, T. Kamphans, G. Nadav, N. Fleischer, H. Fröhlich, P. Krawitz, CADA: phenotype-driven gene prioritization based on a case-enriched knowledge graph. NAR Genom Bioinform. 3, lqab078 (2021).
4. A. Thormann, M. Halachev, W. McLaren, D. J. Moore, V. Svinti, A. Campbell, S. M. Kerr, M. Tischkowitz, S. E. Hunt, M. G. Dunlop, M. E. Hurles, C. F. Wright, H. V. Firth, F. Cunningham, D. R. FitzPatrick, Flexible and scalable diagnostic filtering of genomic variants using G2P with Ensembl VEP. Nat. Commun. 10, 2373 (2019).
5. D. N. Louden, MedGen: NCBI's Portal to Information on Medical Conditions with a Genetic Component. Med. Ref. Serv. Q. 39, 183-191 (2020).
6. M. J. Landrum, S. Chitipiralla, G. R. Brown, C. Chen, B. Gu, J. Hart, D. Hoffman, W. Jang, K. Kaur, C. Liu, V. Lyoshin, Z. Maddipatla, R. Maiti, J. Mitchell, N. O'Leary, G. R. Riley, W. Shi, G. Zhou, V. Schneider, D. Maglott, J. B. Holmes, B. L. Kattman, ClinVar: improvements to accessing data. Nucleic Acids Res. 48, D835-D844 (2020).
7. A. Allot, Y. Peng, C.-H. Wei, K. Lee, L. Phan, Z. Lu, LitVar: a semantic search engine for linking genomic variant data in PubMed and PMC. Nucleic Acids Res. 46, W530-W536 (2018).
8. M. Jacomy, T. Venturini, S. Heymann, M. Bastian, ForceAtlas2, a continuous graph layout algorithm for handy network visualization designed for the Gephi software. PLoS One. 9, e98679 (2014).
9. Z. Chen, Y. Zheng, Y. Yang, Y. Huang, S. Zhao, H. Zhao, C. Yu, X. Dong, Y. Zhang, L. Wang, Z. Zhao, S. Wang, Y. Yang, Y. Ming, J. Su, G. Qiu, Z. Wu, T. J. Zhang, N. Wu, PhenoApt leverages clinical expertise to prioritize candidate genes via machine learning. Am. J. Hum. Genet. 109, 270-281 (2022).
10. M. Zhao, J. M. Havrilla, L. Fang, Y. Chen, J. Peng, C. Liu, C. Wu, M. Sarmady, P. Botas, J. Isla, G. J. Lyon, C. Weng, K. Wang, Phen2Gene: rapid phenotype-driven gene prioritization for rare diseases. NAR Genom Bioinform. 2, lqaa032 (2020).
11. P. N. Robinson, V. Ravanmehr, J. O. B. Jacobsen, D. Danis, X. A. Zhang, L. C. Carmody, M. A. Gargano, C. L. Thaxton, UNC Biocuration Core, G. Karlebach, J. Reese, M. Holtgrewe, S. Köhler, J. A. McMurry, M. A. Haendel, D. Smedley, Interpretable Clinical Genomics with a Likelihood Ratio Paradigm. Am. J. Hum. Genet. 107, 403-417 (2020).

### General References

1. Dewey, F. E. et al. Distribution and clinical impact of functional variants in 50,726 whole-exome sequences from the DiscovEHR study. Science 354, (2016).
2. Robinson, P. N. Deep phenotyping for precision medicine. Human Mutation vol. 33 777-780 Preprint at https://doi.org/10.1002/humu.22080 (2012).
3. Köhler, S., Kindle, G. & Robinson, P. N. The Human Phenotype Ontology in 2021. (2021).
4. Haendel, M. A., Chute, C. G. & Robinson, P. N. Classification, Ontology, and Precision Medicine. N. Engl. J. Med. 379, 1452-1462 (2018).
5. Seo, G. H. et al. Diagnostic yield and clinical utility of whole exome sequencing using an automated variant prioritization system, EVIDENCE. Clin. Genet. 98, 562-570 (2020).
6. Trujillano, D. et al. Clinical exome sequencing: results from 2819 samples reflecting 1000 families. Eur. J. Hum. Genet. 25, 176-182 (2017).
7. Peng, C. et al. CADA: phenotype-driven gene prioritization based on a case-enriched knowledge graph. NAR Genom Bioinform 3, lqab078 (2021).
8. Osmond, M. et al. PhenomeCentral: 7 years of rare disease matchmaking. Hum. Mutat. 43, 674-681 (2022).
9. Thormann, A. et al. Flexible and scalable diagnostic filtering of genomic variants using G2P with Ensembl VEP. Nat. Commun. 10, 2373 (2019).
10. Amberger, J. S., Bocchini, C. A., Schiettecatte, F., Scott, A. F. & Hamosh, A. OMIM.org: Online Mendelian Inheritance in Man (OMIM®), an online catalog of human genes and genetic disorders. Nucleic Acids Res. 43, D789-98 (2015).
11. Louden, D. N. MedGen: NCBI's Portal to Information on Medical Conditions with a Genetic Component. Med. Ref. Serv. Q. 39, 183-191 (2020).
12. Lee, D. D. & Seung, H. S. Learning the parts of objects by non-negative matrix factorization. Nature 401, 788-791 (1999).
13. Röder, M., Both, A. & Hinneburg, A. Exploring the space of topic coherence measures. in Proceedings of the Eighth ACM International Conference on Web Search and Data Mining - WSDM '15 (ACM Press, 2015). doi: 10.1145/2684822.2685324.
14. McInnes, L., Healy, J., Saul, N. & Großberger, L. UMAP: Uniform Manifold Approximation and Projection. Journal of Open Source Software vol. 3 861 Preprint at https://doi.org/10.21105/joss.00861 (2018).
15. Chen, Z. et al. PhenoApt leverages clinical expertise to prioritize candidate genes via machine learning. Am. J. Hum. Genet. 109, 270-281 (2022).
16. Zhao, M. et al. Phen2Gene: rapid phenotype-driven gene prioritization for rare diseases. NAR Genom Bioinform 2, lqaa032 (2020).
17. Robinson, P. N. et al. Interpretable Clinical Genomics with a Likelihood Ratio Paradigm. Am. J. Hum. Genet. 107, 403-417 (2020).
18. 100,000 Genomes Project Pilot Investigators et al. 100,000 Genomes Pilot on Rare-Disease Diagnosis in Health Care - Preliminary Report. N. Engl. J. Med. 385, 1868-1880 (2021).
19. Larkin, J., McDermott, J., Simon, D. P. & Simon, H. A. Expert and novice performance in solving physics problems. Science 208, 1335-1342 (1980).
20. Coderre, S., Mandin, H., Harasym, P. H. & Fick, G. H. Diagnostic reasoning strategies and diagnostic success. Med. Educ. 37, 695-703 (2003).
21. Shen, F. et al. HPO2Vec+: Leveraging heterogeneous knowledge resources to enrich node embeddings for the Human Phenotype Ontology. J. Biomed. Inform. 96, 103246 (2019).
22. Tiddi, I. & Schlobach, S. Knowledge graphs as tools for explainable machine learning: A survey. Artif. Intell. 302, 103627 (2022).
23. Zhang, Y. et al. High-throughput phenotyping with electronic medical record data using a common semi-supervised approach (PheCAP). Nat. Protoc. 14, 3426-3444 (2019).
24. Jacobsen, J. O. B. et al. The GA4GH Phenopacket schema defines a computable representation of clinical data. Nat. Biotechnol. 40, 817-820 (2022).
25. Yurkovich, J. T., Tian, Q., Price, N. D. & Hood, L. A systems approach to clinical oncology uses deep phenotyping to deliver personalized care. Nat. Rev. Clin. Oncol. 17, 183-194 (2020).
26. Chute, C. G. Clinical Data Retrieval and Analysis. I've Seen a Case Like That Before. Annals of the New York Academy of Sciences vol. 670 133-140 Preprint at https://doi.org/10.1111/j.1749-6632.1992.tb26083.x (1992).

## Claims

1. A computer-implemented method for decision support of a user to standardize phenotyping in genomic analysis of a subject, wherein the method comprises:
- a) receiving a list of symptoms comprising at least one symptom observed for the subject, wherein the list of symptoms comprises an integer number N of symptoms;
- if the integer number N is higher than a threshold value, the method further comprises a step b1) of receiving a first graph comprising nodes and weighted links, wherein:
o each node is representative of one symptom, each symptom being associated to at least one gene;
o each link connecting two nodes is representative of a similarity between the two symptoms associated to the two connected nodes, and
o the weight of each link represents a degree of similarity between said two symptoms associated to the two connected nodes;
said first graph being previously obtained calculating symptoms pair similarities using information concerning associations between at least one symptom and at least one gene considered to be responsible of said associated at least one symptom;
- else if the integer number is lower than or equal to said threshold value, the method further comprises b21), b22), b23) and b24) comprising:
- b21) receiving a second graph being previously obtained applying a matrix factorization to a gene-symptom matrix, said second graph comprising nodes and weighted links, wherein each node is representative of one symptom, each symptom being associated to at least one gene; each node is associated to at least one group of nodes of the second graph; and each node is associated to at least one score of specificity, each score of specificity representing the specificity of the symptom associated to the node with respect to the symptoms of the nodes in each group to which is associated said node; wherein said gene-symptom matrix is representative of associations between at least one symptom and at least one gene considered to be responsible of said associated at least one symptom, wherein each coefficient *aᵢⱼ* of said gene-symptom matrix represents a probability of association between gene *i* and symptom *j*;
wherein each gene represented in the gene-symptom matrix is associated to each group of nodes of the second graph by a gene-group weighting score;
- b22) evaluating the specificity of the received list of symptoms, using the score of specificity of the node associated to the at least one symptom in the second graph, and if the specificity of the list of symptoms does not satisfy a predefined rule, providing to the user a suggestion of at least one new symptom; each at least one suggested new symptom being obtained as the symptom associated to the node in the first graph connected by one link to the node associated to the at least one symptom of the received list of symptoms;
- b23) if the at least one suggested new symptom is added to the received list of symptoms by the user, evaluating the specificity of the updated list of symptoms comprising the at least one received symptom and the at least one new suggested symptom, using the score of specificity of the node in the second graph associated to the symptoms in the updated list of symptoms;
- b24) whenever the specificity of the list of symptoms satisfies the predefined rule, using said list of symptoms and the first graph to obtain at least one gene having the highest probability to be associated to the list of symptoms;
the method further comprising the following steps:
- c) if, after providing to the user a suggestion of at least one new symptom, the specificity of the updated list of symptoms does not satisfy the predefined rule:
• generating a symptoms binary vector representing the symptoms in the list of symptoms and applying the matrix factorization to said binary vector so as to obtain a representation of the updated list of symptoms on the groups of the second graph, said representation comprising one patient score for each group of the second graph;
• calculating a difference between each patient score and each gene-group weighting score for each group of the second graph;
• normalizing said differences with respect to a reference set of distances representative of a subject without symptoms;
• selecting at least one gene having the highest probability to be associated to the updated list of symptoms as the at least one gene having the highest normalized difference;
- d) outputting the at least one gene associated to the list of symptoms or the updated list of symptoms.

2. A device for decision support of a user to standardize phenotyping in genomic analysis of a subject, wherein the device comprises:
- at least one input configured to receive:
o a list of symptoms comprising at least one symptom observed for the subject, wherein the list of symptoms comprises an integer number N of symptoms;
o a first graph comprising nodes and weighted links, wherein each node is representative of one symptom, each symptom being associated to at least one gene; each link connecting two nodes is representative of a similarity between the two symptoms associated to the two connected nodes, and the weight of each link represents a degree of similarity between said two symptoms associated to the two connected nodes;
said first graph being previously obtained calculating symptoms pair similarities using information concerning associations between at least one symptom and at least one gene considered to be responsible of said associated at least one symptom;
o a second graph being previously obtained by applying a matrix factorization to a gene-symptom matrix, said second graph comprising nodes and weighted links, wherein each node is representative of one symptom, each symptom being associated to at least one gene; each node is associated to at least one group of nodes of the second graph; and each node is associated to at least one score of specificity, each score of specificity representing the specificity of the symptom associated to the node with respect to the symptoms of the nodes in each group to which is associated said node; wherein said gene-symptom matrix is representative of associations between at least one symptom and at least one gene considered to be responsible of said associated at least one symptom, wherein each coefficient *aᵢⱼ* of said gene-symptom matrix represents a probability of association between gene *i* and symptom *j*; and wherein each gene represented in the gene-symptom matrix is associated to each group of nodes of the second graph by a gene-group weighting score;
- at least one processor configured to:
o if the integer number N is less than or equal to a threshold value:
▪ evaluate a specificity of the list of symptoms, using the score of specificity of the node associated the at least one symptom in the second graph, and if the specificity of the list of symptoms does not satisfy a predefined rule, providing to the user a suggestion of at least one symptom, each at least one suggested symptom being obtained as the symptom associated to the node in the first graph connected by one link to the node associated to the at least one received symptom;
▪ if the at least one suggested symptom is added to the list of symptoms by the user, evaluate the specificity of the list of symptoms comprising the at least one received symptom and the at least one suggested symptom, using the score of specificity of the node in the second graph associated to the symptoms in the list of symptoms;
▪ whenever the specificity of the list of symptoms satisfies the predefined rule, use said list of symptoms and the first graph to obtain at least one gene having the highest probability to be associated to the list of symptoms;
o if, after providing to the user a suggestion of at least one symptom, the specificity of the list of symptoms does not satisfy a predefined rule:
▪ generate a symptoms binary vector representing the symptoms in the list of symptoms and applying the matrix factorization to said binary vector so as to obtain a representation of the list of symptoms on the groups of the second graph, said representation comprising one patient score for each group of the second graph;
▪ calculate a difference between each patient score and each gene-group weighting score for each group of the second graph;
▪ normalize said differences with respect to a reference set of distances representative of a subject without symptoms;
▪ select at least one gene having the highest probability to be associated to the list of symptoms as the at least one gene having the highest normalized difference;
- at least one output configured to provide the at least one gene associated to the list of symptoms.

3. The device according to claim 2, wherein the matrix factorization is a non-negative matrix factorization.

4. The device according to claim 3, wherein the non-negative matrix factorization applied to the gene-symptom matrix is a Non-negative Double Singular Value Decomposition (NNDSVD) initialization.

5. The device according to any one of claims 2 to 4, wherein information concerning associations between at least one symptom and at least one gene is a gene-symptom list representative of associations between at least one symptom and at least one gene considered to be responsible of said associated at least one symptom or is the gene-symptom matrix.

6. The device according to any one of claims 2 to 5, wherein the weight of each link in the first graph is determined with a node similarity algorithm.

7. The device according to any one of claims 2 to 6, wherein using the list of symptoms and the first graph to obtain the at least one gene having the highest probability to be associated to the list of symptoms comprises:
a. determining at least one additional symptom associated to at least one symptom of the list of symptoms by identifying, among the nodes of the first graph, at least one node connected, by a weighted link having a weight higher than a predefined threshold, to one of the at least one node corresponding to the symptoms on the list of symptoms;
b. calculating a subject specific matrix comprising as columns each column of the gene-phenotype matrix for the symptom *j* associated to the at least one symptom of the subject comprised in the list of symptoms and said at least one additional symptom;
c. calculating a score vector, wherein each coefficient *bᵢ* of the score vector is obtained as function of each row coefficients of the subject specific matrix so as to be representative of the probability that each gene of the subject specific matrix is associated to the symptoms observed for the subject;
d. using the score vector to obtain an information on the at least one gene that is more likely to be associated to the symptoms observed for the subject.

8. The device according to any one of claims 2 to 7, wherein, the at least one processor is further configured to rank the genes associated to the symptoms observed in the subject on the base of the base of the score vector.

## Patentansprüche

1. Computerimplementiertes Verfahren zur Entscheidungsunterstützung eines Benutzers zur Standardisierung der Phänotypisierung bei der genomischen Analyse eines Probanden, wobei das Verfahren Folgendes umfasst:
- a) Empfangen einer Liste von Symptomen, die mindestens ein für den Probanden beobachtetes Symptom umfasst, wobei die Liste der Symptome eine ganze Zahl N von Symptomen umfasst;
- wenn die ganze Zahl N höher als ein Schwellenwert ist, umfasst das Verfahren ferner einen Schritt b1) des Empfangens eines ersten Graphen, der Knoten und gewichtete Verknüpfungen umfasst, wobei:
o jeder Knoten repräsentativ für ein Symptom ist, wobei jedes Symptom mindestens einem Gen zugeordnet ist;
o jede Verknüpfung, die zwei Knoten verbindet, repräsentativ für eine Ähnlichkeit zwischen den beiden Symptomen ist, die den beiden verbundenen Knoten zugeordnet sind, und
o das Gewicht jeder Verknüpfung ein Maß an Ähnlichkeit zwischen den beiden Symptomen darstellt, die den beiden verbundenen Knoten zugeordnet sind;
wobei der erste Graph zuvor durch Berechnen von Ähnlichkeiten zwischen Symptompaaren unter Verwendung von Informationen über Zuordnungen zwischen mindestens einem Symptom und mindestens einem Gen, das als verantwortlich für das damit verbundene mindestens eine Symptom angesehen wird, erhalten wurde;
- andernfalls, wenn die ganze Zahl kleiner oder gleich dem Schwellenwert ist, das Verfahren ferner b21), b22), b23) und b24) umfasst, umfassend:
- b21) Empfangen eines zweiten Graphen, der zuvor durch Anwenden einer Matrixfaktorisierung auf eine Gen-Symptom-Matrix erhalten wurde, wobei der zweite Graph Knoten und gewichtete Verknüpfungen umfasst, wobei jeder Knoten für ein Symptom repräsentativ ist, wobei jedes Symptom mindestens einem Gen zugeordnet ist; jeder Knoten ist mindestens einer Gruppe von Knoten des zweiten Graphen zugeordnet; und jeder Knoten ist mindestens einem Spezifitätspunktwert zugeordnet, wobei jeder Spezifitätspunktwert die Spezifität des dem Knoten zugeordneten Symptoms in Bezug auf die Symptome der Knoten in jeder Gruppe, der der Knoten zugeordnet ist, darstellt; wobei die Gen-Symptom-Matrix repräsentativ für Zuordnungen zwischen mindestens einem Symptom und mindestens einem Gen ist, das als verantwortlich für das damit verbundene mindestens eine Symptom angesehen wird, wobei jeder Koeffizient *aᵢⱼ* der Gen-Symptom-Matrix eine Wahrscheinlichkeit einer Zuordnung zwischen Gen *i* und Symptom *j* darstellt;
wobei jedes in der Gen-Symptom-Matrix dargestellte Gen jeder Gruppe von Knoten des zweiten Graphen durch einen Gengruppengewichtungspunktwert zugeordnet ist;
- b22) Bewerten der Spezifität der empfangenen Liste von Symptomen unter Verwendung des Spezifitätspunktwerts des Knotens, der dem mindestens einen Symptom in dem zweiten Graphen zugeordnet ist, und wenn die Spezifität der Liste von Symptomen eine vordefinierte Regel nicht erfüllt, Bereitstellen eines Vorschlags für mindestens ein neues Symptom an den Benutzer; wobei jedes mindestens eine vorgeschlagene neue Symptom als das Symptom erhalten wird, das dem Knoten in dem ersten Graph zugeordnet ist, der durch eine Verknüpfung mit dem Knoten verbunden ist, der dem mindestens einen Symptom der empfangenen Symptomliste zugeordnet ist;
- b23) wenn das mindestens eine vorgeschlagene neue Symptom von dem Benutzer zu der empfangenen Liste von Symptomen hinzugefügt wird, die Spezifität der aktualisierten Liste von Symptomen, die das mindestens eine empfangene Symptom und das mindestens eine neue vorgeschlagene Symptom umfasst, unter Verwendung des Punktwerts der Spezifität des Knotens in dem zweiten Graph, der den Symptomen in der aktualisierten Liste von Symptomen zugeordnet ist;
- b24) immer dann, wenn die Spezifität der Liste der Symptome die vordefinierte Regel erfüllt, wobei die Liste der Symptome und der erste Graph verwendet werden, um mindestens ein Gen zu erhalten, das mit der höchsten Wahrscheinlichkeit der Liste der Symptome zugeordnet werden kann;
wobei das Verfahren ferner folgende Schritte umfasst:
- c) wenn die Spezifität der aktualisierten Symptomliste, nachdem dem Benutzer ein Vorschlag für mindestens ein neues Symptom vorgelegt wurde, nicht der vordefinierten Regel entspricht:
• Erzeugen eines Symptom-Binärvektors, der die Symptome in der Liste der Symptome darstellt, und Anwenden der Matrixfaktorisierung auf den Binärvektor, um eine Darstellung der aktualisierten Liste der Symptome auf den Gruppen des zweiten Graphen zu erhalten, wobei die Darstellung einen Patientenpunktwert für jede Gruppe des zweiten Graphen umfasst;
• Berechnen einer Differenz zwischen jedem Patientenpunktwert und jedem Gengruppengewichtungspunktwert für jede Gruppe des zweiten Graphen;
• Normalisieren dieser Differenzen in Bezug auf einen Referenzsatz von Entfernungen, der für einen Probanden ohne Symptome repräsentativ ist;
• Auswählen von mindestens einem Gen mit der höchsten Wahrscheinlichkeit, der aktualisierten Liste von Symptomen zugeordnet zu werden, als dem mindestens einen Gen mit der höchsten normalisierten Differenz;
- d) Ausgeben des mindestens einen Gens, das der Liste der Symptome oder der aktualisierten Liste der Symptome zugeordnet ist.

2. Vorrichtung zur Entscheidungsunterstützung eines Benutzers zur Standardisierung der Phänotypisierung bei der genomischen Analyse eines Probanden, wobei die Vorrichtung Folgendes umfasst:
- mindestens einen Eingang, der so konfiguriert ist, dass er Folgendes empfängt:
o eine Liste von Symptomen, die mindestens ein für den Probanden beobachtetes Symptom umfasst, wobei die Liste der Symptome eine ganze Zahl N von Symptomen umfasst;
o wobei ein erster Graph Knoten und gewichtete Verknüpfungen umfasst, wobei jeder Knoten für ein Symptom repräsentativ ist, wobei jedes Symptom mindestens einem Gen zugeordnet ist; wobei jede Verknüpfung, die zwei Knoten verbindet, repräsentativ ist für eine Ähnlichkeit zwischen den beiden Symptomen, die den beiden verbundenen Knoten zugeordnet sind, und das Gewicht jeder Verknüpfung einen Grad der Ähnlichkeit zwischen den beiden Symptomen, die den beiden verbundenen Knoten zugeordnet sind, darstellt;
wobei der erste Graph zuvor durch Berechnen von Ähnlichkeiten zwischen Symptompaaren unter Verwendung von Informationen über Zuordnungen zwischen mindestens einem Symptom und mindestens einem Gen, das als verantwortlich für das damit verbundene mindestens eine Symptom angesehen wird, erhalten wurde;
o wobei ein zweiter Graph zuvor durch Anwenden einer Matrixfaktorisierung auf eine Gen-Symptom-Matrix erhalten wurde, der zweite Graph umfassend Knoten und gewichtete Verknüpfungen, wobei jeder Knoten für ein Symptom repräsentativ ist, wobei jedes Symptom mindestens einem Gen zugeordnet ist; jeder Knoten mindestens einer Gruppe von Knoten des zweiten Graphen zugeordnet ist; und jeder Knoten mindestens einem Spezifitätspunktwert zugeordnet ist, wobei jeder Spezifitätspunktwert die Spezifität des dem Knoten zugeordneten Symptoms in Bezug auf die Symptome der Knoten in jeder Gruppe, der der Knoten zugeordnet ist, darstellt; wobei die Gen-Symptom-Matrix repräsentativ für Zuordnungen zwischen mindestens einem Symptom und mindestens einem Gen ist, das für das betreffende zugeordnete mindestens eine Symptom verantwortlich ist, wobei jeder Koeffizient *aᵢⱼ* der Gen-Symptom-Matrix eine Wahrscheinlichkeit der Zuordnung zwischen Gen i und Symptom *j* darstellt; und wobei jedes in der Gen-Symptom-Matrix repräsentierte Gen jeder Gruppe von Knoten des zweiten Graphen durch einen Gengruppengewichtungspunktwert zugeordnet ist;
- mindestens einen Prozessor, der für Folgendes konfiguriert ist:
o wenn die ganze Zahl N kleiner oder gleich einem Schwellenwert ist:
▪ Bewerten einer Spezifität der Liste der Symptome unter Verwendung des Spezifitätspunktwerts des Knotens, der dem mindestens einen Symptom in dem zweiten Graph zugeordnet ist, und wenn die Spezifität der Liste der Symptome keine vordefinierte Regel erfüllt, Bereitstellen eines Vorschlags für mindestens ein Symptom an den Benutzer, wobei jedes mindestens eine vorgeschlagene Symptom als das Symptom erhalten wird, das dem Knoten in dem ersten Graph zugeordnet ist, der durch eine Verknüpfung mit dem Knoten verbunden ist, der dem mindestens einen empfangenen Symptom zugeordnet ist;
▪ wenn das mindestens eine vorgeschlagene Symptom von dem Benutzer zu der Liste der Symptome hinzugefügt wird, die Spezifität der Liste der Symptome bewerten, die das mindestens eine empfangene Symptom und das mindestens eine vorgeschlagene Symptom umfasst, unter Verwendung des Punktwerts der Spezifität des Knotens in dem zweiten Graph, der den Symptomen in der Liste der Symptome zugeordnet ist;
▪ wenn die Spezifität der Liste der Symptome die vordefinierte Regel erfüllt, diese Liste der Symptome und den ersten Graph verwenden, um mindestens ein Gen mit der höchsten Wahrscheinlichkeit zu erhalten, der Liste der Symptome zugeordnet zu werden;
o wenn nach Vorschlagen mindestens eines Symptoms an den Benutzer die Spezifität der Symptomliste einer vordefinierten Regel nicht entspricht:
▪ Erzeugen eines Symptom-Binärvektors, der die Symptome in der Liste der Symptome darstellt, und Anwenden der Matrixfaktorisierung auf den Binärvektor, um eine Darstellung der Liste der Symptome auf den Gruppen des zweiten Graphen zu erhalten, wobei die Darstellung einen Patientenpunktwert für jede Gruppe des zweiten Graphen umfasst;
▪ Berechnen einer Differenz zwischen jedem Patientenpunktwert und jedem Gengruppengewichtungspunktwert für jede Gruppe des zweiten Graphen;
▪ Normalisieren dieser Differenzen in Bezug auf eine Referenzmenge von Entfernungen, die für einen Probanden ohne Symptome repräsentativ sind;
▪ Auswählen mindestens eines Gens mit der höchsten Wahrscheinlichkeit, der Liste der Symptome zugeordnet zu werden, als das mindestens eine Gen mit der höchsten normalisierten Differenz;
- mindestens einen Ausgang, der konfiguriert ist, um das mindestens eine Gen bereitzustellen, das der Liste der Symptome zugeordnet ist.

3. Vorrichtung nach Anspruch 2, wobei die Matrixfaktorisierung eine nicht-negative Matrixfaktorisierung ist.

4. Vorrichtung nach Anspruch 3, wobei die nicht-negative Matrixfaktorisierung, die auf die Gen-Symptom-Matrix angewendet wird, eine Nicht-negative Double Singular Value Decomposition (NNDSVD)-Initialisierung ist.

5. Vorrichtung nach einem der Ansprüche 2 bis 4, wobei Informationen über Zuordnungen zwischen mindestens einem Symptom und mindestens einem Gen eine Gen-Symptom-Liste ist, die repräsentativ für Zuordnungen zwischen mindestens einem Symptom und mindestens einem Gen ist, das als verantwortlich für das zugeordnete mindestens eine Symptom angesehen wird, oder die Gen-Symptom-Matrix ist.

6. Vorrichtung nach einem der Ansprüche 2 bis 5, wobei das Gewicht jeder Verknüpfung in dem ersten Graph mit einem Knotenähnlichkeitsalgorithmus bestimmt wird.

7. Vorrichtung nach einem der Ansprüche 2 bis 6, wobei die Verwendung der Symptomliste und des ersten Graphen zum Erhalten des mindestens einen Gens mit der höchsten Wahrscheinlichkeit, der Symptomliste zugeordnet zu werden, Folgendes umfasst:
a. Bestimmen von mindestens einem zusätzlichen Symptom, das mindestens einem Symptom der Symptomliste zugeordnet ist, indem unter den Knoten des ersten Graphen mindestens ein Knoten identifiziert wird, der durch eine gewichtete Verknüpfung mit einem Gewicht, das einen vordefinierten Schwellenwert überschreitet, mit einem der mindestens einen Knoten verbunden ist, die den Symptomen in der Symptomliste entsprechen;
b. Berechnen einer probandenspezifischen Matrix, die als Spalten jede Spalte der Gen-Phänotyp-Matrix für das Symptom j umfasst, das dem mindestens einen Symptom des in der Symptomliste enthaltenen Probanden und dem mindestens einen zusätzlichen Symptom zugeordnet ist;
c. Berechnen eines Punktwertvektors, wobei jeder Koeffizient *bᵢ* des Punktwertvektors als Funktion jeder Reihenkoeffizienten der probandenspezifischen Matrix erhalten wird, um repräsentativ für die Wahrscheinlichkeit zu sein, dass jedes Gen der probandenspezifischen Matrix den für den Probanden beobachteten Symptomen zugeordnet ist;
d. Verwenden des Punktwertvektors, um Informationen über mindestens ein Gen zu erhalten, das mit größerer Wahrscheinlichkeit den bei dem Probanden beobachteten Symptomen zugeordnet wird.

8. Vorrichtung nach einem der Ansprüche 2 bis 7, wobei der mindestens eine Prozessor ferner so konfiguriert ist, dass er die Gene, die den beim Probanden beobachteten Symptomen zugeordnet sind, basierend auf der Basis des Punktwertvektors einstuft.

## Revendications

1. Une méthode mise en œuvre par ordinateur pour l'aide à la décision d'un utilisateur pour standardiser le phénotypage dans l'analyse génomique d'un sujet, ladite méthode comprend :
- a) la réception d'une liste de symptômes comprenant au moins un symptôme observé chez le sujet, dans laquelle la liste de symptômes comprend un nombre entier N de symptômes ;
- si le nombre entier N est supérieur à une valeur seuil, la méthode comprend en outre une étape b1) de réception d'un premier graphe comprenant des nœuds et des liens pondérés, dans lequel :
o chaque nœud est représentatif d'un symptôme, chaque symptôme étant associé à au moins un gène ;
o chaque lien reliant deux nœuds est représentatif d'une similarité entre les deux symptômes associés aux deux nœuds connectés, et
o le poids de chaque lien représente un degré de similarité entre lesdits deux symptômes associés aux deux nœuds connectés ;
ledit premier graphe étant préalablement obtenu par calcul des similarités par paires de symptômes en utilisant des informations concernant les associations entre au moins un symptôme et au moins un gène considéré comme responsable dudit au moins un symptôme ;
- sinon, si ledit nombre entier est inférieur ou égal à ladite valeur seuil, la méthode comprend en outre b21), b22), b23) et b24), comprenant :
- b21) la réception d'un second graphe étant obtenu préalablement par application d'une factorisation de matrice à une matrice gène-symptôme, ledit second graphe comprenant des nœuds et des liens pondérés, dans lequel chaque nœud est représentatif d'un symptôme, chaque symptôme étant associé à au moins un gène ; chaque nœud est associé à au moins un groupe de nœuds du second graphe ; et chaque nœud est associé à au moins un score de spécificité, chaque score de spécificité représentant la spécificité du symptôme associé au nœud par rapport aux symptômes des nœuds dans chacun des groupes auxquels ledit nœud est associé ; dans lequel ladite matrice gène-symptôme est représentative des associations entre au moins un symptôme et au moins un gène considéré comme étant responsable dudit au moins un symptôme associé, dans laquelle chaque coefficient *aᵢⱼ* de ladite matrice gène-symptôme représente une probabilité d'association entre le gène *i* et le symptôme *j* ;
dans laquelle chaque gène représenté dans la matrice gène-symptôme est associé à chaque groupe de nœuds du second graphe par un score de pondération gène-groupe ;
- b22) l'évaluation de la spécificité de la liste de symptômes reçue, en utilisant le score de spécificité du nœud associé au au moins un symptôme dans le second graphe, et, si la spécificité de la liste de symptômes ne satisfait pas une règle prédéfinie, fourniture à l'utilisateur une suggestion d'au moins un nouveau symptôme, chacun des au moins un nouveau symptôme suggéré étant obtenu comme le symptôme associé au nœud dans le premier graphe relié par un lien au nœud associé au au moins un symptôme de la liste reçue ;
- b23) si le au moins un nouveau symptôme suggéré est ajouté à la liste reçue par l'utilisateur, l'évaluation de la spécificité de la liste de symptômes mise à jour comprenant le au moins un symptôme reçu et le au moins un nouveau symptôme suggéré, en utilisant le score de spécificité du nœud du second graphe associé aux symptômes de ladite liste de symptômes mise à jour ;
- b24) lorsque la spécificité de la liste de symptômes satisfait la règle prédéfinie, l'utilisation de ladite liste de symptômes et du premier graphe pour obtenir au moins un gène ayant la probabilité la plus élevée d'être associé à la liste de symptômes ;
la méthode comprenant en outre les étapes suivantes :
- c) si, après avoir fourni à l'utilisateur une suggestion d'au moins un nouveau symptôme, la spécificité de la liste de symptômes mise à jour ne satisfait pas la règle prédéfinie :
o générer un vecteur binaire de symptômes représentant les symptômes de la liste de symptômes et appliquer la factorisation de matrice audit vecteur binaire afin d'obtenir une représentation de la liste de symptômes mise à jour sur les groupes du second graphe, ladite représentation comprenant un score patient pour chaque groupe du second graphe ;
o calculer une différence entre chaque score patient et chaque score de pondération gène-groupe pour chaque groupe du second graphe ;
o normaliser lesdites différences par rapport à un ensemble de distances de référence représentatives d'un sujet sans symptômes ;
o sélectionner au moins un gène ayant la probabilité la plus élevée d'être associé à la liste de symptômes mise à jour en tant que le au moins un gène ayant la plus grande différence normalisée ;
- d) fournir en sortie le au moins un gènes associé à la liste de symptômes ou à la liste de symptômes mise à jour.

2. Un dispositif d'aide à la décision d'un utilisateur pour standardiser le phénotypage dans l'analyse génomique d'un sujet, dans lequel le dispositif comprend :
- au moins une entrée configurée pour recevoir :
o une liste de symptômes comprenant au moins un symptôme observé chez le sujet, dans laquelle la liste de symptômes comprend un nombre entier N de symptômes ;
o un premier graphe comprenant des nœuds et des liens pondérés, dans lequel chaque nœud est représentatif d'un symptôme, chaque symptôme étant associé à au moins un gène ; chaque lien reliant deux nœuds est représentatif d'une similarité entre les deux symptômes associés aux deux nœuds reliés, et le poids de chaque lien représente un degré de similarité entre lesdits deux symptômes associés aux deux nœuds reliés ;
ledit premier graphe étant préalablement obtenu par calcul des similarités par paires de symptômes en utilisant des informations concernant les associations entre au moins un symptôme et au moins un gène considéré comme étant responsable dudit symptôme associé au au moins un symptôme ;
o un second graphe obtenu préalablement par application d'une factorisation de matrice à une matrice gène-symptôme, ledit second graphe comprenant des nœuds et des liens pondérés, dans lequel chaque nœud est représentatif d'un symptôme, chaque symptôme étant associé à au moins un gène ; chaque nœud est associé à au moins un groupe de nœuds du second graphe ; et chaque nœud est associé à au moins un score de spécificité, chaque score de spécificité représentant la spécificité du symptôme associé au nœud par rapport aux symptômes des nœuds dans chaque groupe auquel est associé ledit nœud ; dans lequel ladite matrice gène-symptôme est représentative des associations entre au moins un symptôme et au moins un gène considéré comme étant responsable dudit symptôme associé, dans lequel chaque coefficient *aᵢⱼ* de ladite matrice représente une probabilité d'association entre le gène *i* et le symptôme *j* ; et dans lequel chaque gène représenté dans ladite matrice gène-symptôme est associé à chaque groupe de nœuds du second graphe par un score de pondération gène-groupe ;
- au moins un processeur configuré pour :
o si le nombre entier N est inférieur ou égal à une valeur seuil :
▪ évaluer une spécificité de la liste de symptômes, en utilisant le score de spécificité du nœud associé au au moins un symptôme dans le second graphe, et si la spécificité de la liste ne satisfait pas une règle prédéfinie, fournir à l'utilisateur une suggestion d'au moins un symptôme, chaque symptôme suggéré étant obtenu comme le symptôme associé au nœud du premier graphe relié par un lien au nœud associé au au moins un symptôme reçu ;
▪ si le ou les symptômes suggérés sont ajoutés à la liste de symptômes par l'utilisateur, évaluer la spécificité de la liste de symptômes comprenant au moins un symptôme reçu et au moins un symptôme suggéré, en utilisant le score de spécificité du nœud du second graphe associé aux symptômes de la liste de symptômes ;
▪ chaque fois que la spécificité de la liste de symptômes satisfait la règle prédéfinie, utiliser ladite liste de symptômes et le premier graphe pour obtenir au moins un gène ayant la probabilité la plus élevée d'être associé à ladite liste de symptômes;
o si, après avoir fourni à l'utilisateur au moins un symptôme, la spécificité de la liste ne satisfait pas une règle prédéfinie :
▪ générer un vecteur binaire de symptômes représentant les symptômes de la liste et appliquer la factorisation de matrice audit vecteur afin d'obtenir une représentation de ladite liste de symptômes sur les groupes du second graphe, ladite représentation comprenant un score patient pour chaque groupe ;
▪ calculer une différence entre chaque score patient et chaque score de pondération gène-groupe du second graphe ;
▪ normaliser lesdites différences par rapport à un ensemble de distances de référence représentatives d'un sujet sans symptômes ;
▪ sélectionner au moins un gène ayant la probabilité la plus élevée d'être associé à ladite liste de symptômes en tant que gène ayant la différence normalisée la plus élevée ;
- au moins une sortie configurée pour fournir le au moins un gène associés à la liste de symptômes.

3. Le dispositif selon la revendication 2, dans lequel la factorisation de matrice est une factorisation en matrices non négatives.

4. Le dispositif selon la revendication 3, dans lequel la factorisation en matrices non négatives appliquée à la matrice gène-symptôme est une initialisation par décomposition en valeurs singulières doubles non négatives (NNDSVD).

5. Le dispositif selon l'une quelconque des revendications 2 à 4, dans lequel les informations concernant les associations entre au moins un symptôme et au moins un gène sont une liste gène-symptôme représentative des associations entre au moins un symptôme et au moins un gène considéré comme étant responsable dudit au moins un symptôme associé ou est la matrice gène-symptôme.

6. Le dispositif selon l'une quelconque des revendications 2 à 5, dans lequel le poids de chaque lien dans le premier graphe est déterminé à l'aide d'un algorithme de similarité de nœuds.

7. Le dispositif selon l'une quelconque des revendications 2 à 6, dans lequel l'utilisation de la liste de symptômes et du premier graphe pour obtenir le au moins un gène ayant la probabilité la plus élevée d'être associés à la liste de symptômes comprend :
a. déterminer au moins un symptôme supplémentaire associé au au moins un symptôme de la liste de symptômes en identifiant, parmi les nœuds du premier graphe, au moins un nœud relié, par un lien pondéré ayant un poids plus élevé qu'un seuil prédéfini, à l'un des au moins un nœud correspondant aux symptômes de la liste de symptômes ;
b. calculer une matrice spécifique au sujet comprenant en colonnes chacune des colonnes de la matrice gène-phénotype pour le symptôme *j* associé au au moins un symptôme du sujet compris dans la liste de symptômes et dudit au moins un symptôme supplémentaire ;
c. calculer un vecteur de scores, dans lequel chaque coefficient *bᵢ* du vecteur de scores est obtenu en fonction de chaque coefficient des lignes de la matrice spécifique au sujet de manière à être représentatif de la probabilité que chaque gène de la matrice est associé aux symptômes observés chez le sujet ;
d. utiliser le vecteur de scores pour obtenir une information sur le au moins un sujet gènes qui est le plus susceptible d'être associés aux symptômes observés chez le sujet.

8. Le dispositif selon l'une quelconque des revendications 2 à 7, dans lequel le au moins un processeur est en outre configuré pour classer les gènes associés aux symptômes observés chez le sujet sur la base du vecteur de scores.
